# EUROPEAN PATENT SPECIFICATION

(11) **EP 1 397 380 B1**
(45) Date of publication and mention of the grant of the patent: **27.12.2006**
(21) Application number: 02737552.6
(22) Date of filing: 20.06.2002
(51) Int. Cl.: C07K 14/47

(54) **MULTIPLE ANTIGENIC PEPTIDE DISPLAYING MULTIPLE COPIES OF AN EPITOPE OF A PLAQUE-FORMING POLYPEPTIDE AND METHODS OF USING SAME**
MEHRANTIGENISCHES PEPTID ENTHALTEND MEHRERE KOPIEN EINES EPITOPES VON EINEM ABLAGERUNGSFORMENDEN POLYPEPTID UND DESSEN VERWENDUNG
PRODUIT ANTIGENIQUE COMPRENANT PLUSIEURS COPIES D'UN EPITOPE D'UN POLYPEPTIDE FORMANT DES DEPOTS IMPLIQUE DANS DES MALADIES A FORMATION DE PLAQUES ET PROCEDES D'UTILISATION CORRESPONDANTS

(30) Priority: 20.06.2001 US 299201 P; 12.04.2002 US 371717 P
(43) Date of publication of application: 17.03.2004
(73) Proprietor: Ramot at Tel Aviv University Ltd., 61392 Tel Aviv (IL)
(72) Inventor: SOLOMON, Beka, Herzlia Pituach 46399 (IL)
(74) Representative: Santarelli
(86) International application number: PCT/US2002/019567
(87) International publication number: WO 2003/000719

(56) References cited:
- EP-A- 0 744 467
- EP-A- 0 861 900
- WO-A-01/02861
- DE-A- 19 838 751
- US-A- 5 229 490
- KORTH C ET AL.: "Prion (PrPsc)-specific epitope defined by a monoclonal antibody" NATURE, vol. 390, 6 November 1997 (1997-11-06), pages 74-77, XP002069611
- FRENKEL D ET AL.: "Immunization against Alzheimer's beta-amyloid plaques via EFRH phage administration" PNAS, vol. 97, no. 21, 10 October 2000 (2000-10-10), pages 11455-11459, XP002180061 cited in the application
- HANAN E ET AL.: "Immunomodulation of the Human Prion Peptide 106-126 Aggregation" BIOCHEMICAL AND BIOPHYSICAL RESEARCH COMMUNICATIONS, vol. 280, no. 1, 12 January 2001 (2001-01-12), pages 115-120, XP002246233
- GROSCHUP MH ET AL.: "Antigenic features of prion proteins of sheep and of other mammalian species" JOURNAL OF IMMUNOLOGICAL METHODS, vol. 207, 1997, pages 89-101, XP004093144

## Description

### BACKGROUND OF THE INVENTION

### Field of the Invention

The present invention relates to an antigenic product displaying antigenic peptides for inducing an immune response effective for prevention or reabsorption of deposits of a plaque-forming disease, such as against Aβ in Alzheimer's Disease.

### Description of the Related Art

The pathology of Alzheimer's disease (AD), the most studied conformational disease, is characterized primarily by extracellular amyloid plaques and intracellular neurofibrillary tangles (Price et al., 1993). The relationship between these lesions and the disease process has long been debated. The current dominant theory of AD etiology and pathogenesis is related to the amyloid cascade hypothesis (Hardy and Allsop, 1991; Selkoe, 1996; Hardy, 1997) which states that overproduction of amyloid β peptide (AβP or Aβ), or failure to clear this peptide, leads to AD primarily through amyloid deposition which is involved in the formation of neurofibrillary tangles. These lesions are then associated with cell death which is reflected in memory impairment, the hallmarks of this dementia (Goate, 1991; Hardy et al., 1998). Over a number of years the amyloid cascade hypothesis has gained strength through the observation that AD-causing mutations were identified in the amyloid-β-precursor protein (APP) and in the presenilin genes (Sherrington, et al, 1995; Levy-Lahad, et al., 1995).

Many investigators have studied the propensity of AβP or its fragments to assemble into insoluble aggregates (for review see Maggio and Mantyh, 1996). Whereas the hydrophobic segment in the C-terminal domain of AβP develops a β-strand structure in aqueous solutions, independently of pH or temperature conditions, the N-terminal region can exhibit different conformations and solubility properties depending on environmental conditions (Hollossi et al., 1989, Soto et al., 1995; Barrow and Zagorsky, 1991).

The N-terminal region seems to provide the means for interfibrillary contacts, as well as for interactions between a filament and other proteins or cellular structures that are often associated with β-amyloid depositions (Fraser et al., 1993). The N-terminal domain contains sequences that permit the existence of a dynamic equilibrium between the α-helix and the β-strand conformations. The perturbations of the equilibrium of various conformational states of the β-amyloid peptide can be caused by local pH changes, alterations of environmental hydrophobicity, or binding of other proteins (Soto et al., 1995; Kirschenbaum and Daggett, 1995).

### In vitro disaggregation and prevention of fibrillar β-amylloid

Amyloid filaments, similar to those found in amyloid plaques and cerebrovascular amyloid, can be assembled from chemically synthesized β-peptide under well-defined experimental conditions *in vitro,* and the effect on neural cells may be neurotoxic or neurotrophic, depending on the β-amyloid fibrillar state (Lorenzo and Yanker, 1990; Howlett et al., 1995). *In vitro* amyloid formation is a complex kinetic and thermodynamic process and the reversibility of amyloid plaque growth *in vitro* suggests a steady-state equilibrium between AβP in plaques and in solution (Maggio and Mantyh, 1996). The dependence of AβP polymerization on peptide-peptide interactions to form a β-pleated sheet fibril and the stimulatory influence of other proteins on the reaction suggest that amyloid formation may be subject to modulation.

The essential question is how to prevent or, better, how to reverse the conformational changes that result in the formation of the β-sheet rich pathological protein conformer. Based on the conformational mimicry paradigm, it was hypothesized that short synthetic peptides, "mini chaperones", could be designed to specifically interact with the protein fragment that is undergoing the conformational changes and would be useful in stabilizing a desired conformation by adding specific residues that favor or disfavor the adoption of a particular structural motif (Soto, 1999).

Recently, the immunological approach in the treatment of conformational diseases gained more attention. Antibody-antigen interactions involve conformational changes in both antibody and antigen that can range from insignificant to considerable. Binding of high affinity monoclonal antibodies (mAbs) to regions of high flexibility and antigenicity may alter the molecular dynamics of the whole antigen (Frauenfelder et al., 1979; Karplus and Petsko, 1990).

Appropriate mAbs interact with strategic sites where protein unfolding is initiated, thereby stabilizing the protein and preventing further precipitation (Solomon and Balas, 1991; Katzav-Gozansky et al., 1996). Monoclonal antibodies were found to stabilize the conformation of an antigen against incorrect folding and recognize an incompletely folded epitope, inducing native conformation in a partially unfolded protein (Blond & Goldberg, 1987; Carlson. and Yarmush., 1992; Solomon & Schwartz, 1995).

The laboratory of the present inventors investigated a large panel of mAbs against various regions of AβP and found that only site-directed mAbs toward the N-terminal regions of the β-peptide exhibit anti-aggregating properties, being able to prevent amyloid formation and dissolve already formed aggregates. Mabs 6C6 and 10D5 raised against the N-terminal region of the AβP (residues 1-28) can disaggregate Aβ fibrils and restore the peptide solubility. Binding of such antibodies interfered with noncovalent interactions between the amyloid fibrils and led to deterioration of amyloid fibrillar assembly into an amorphous form, even at molar ratios Ab/peptide 1:10-100. The prevention of peptide aggregation, as well as the solubilization of already formed aggregates, required an equimolar ratio of Ab/peptide, indicating the molecular level of these interactions (Solomon et al., 1996; Hanan and Solomon, 1996; Solomon et al., 1997). The neurotoxicity effect was found to correlate with the formation of Aβ aggregates and with the extent of the β-sheet structure (Pike et al., 1995). The effects of Aβ on MTT reduction on PC 12 cells are known to occur at concentrations below those that result in cell death (Sladowsky et al., 1993) and represent early markers of the metabolic compromise that ultimately leads to cellular degeneration. Binding of mAb 6C6 to fibrillar β-amyloid prevents the neurotoxicity of Aβ, as measured by MTT assay, due to disaggregation of β-amyloid fibrils.

Antibody engineering methods were applied to minimize the size of mAbs (135-900 kDa) while maintaining their biological activity (Winter et al., 1994). These technologies and the application of the PCR technology to create large antibody gene repertoires make antibody phage display a versatile tool for isolation and characterization of single chain Fv (scFv) antibodies (Hoogenboom et al., 1998). The scFvs can be displayed on the surface of the phage for further manipulation or may be released as a soluble scFv (~25 kd) fragment.

The laboratory of the present inventors engineered an scFv which exhibits anti-aggregating properties similar to the parental IgM molecule (Frenkel et al., 2000a). For scFv construction, the antibody genes from the anti-AβP IgM 508 hybridoma were cloned. The secreted antibody showed specific activity toward the AβP molecule in preventing its toxic effects on cultured PC 12 cells.

Site-directed single-chain Fv antibodies are the first step towards targeting therapeutic antibodies into the brain via intracellular or extracellular approaches. The ability of single chain antibody 508F(Fv) to dissolve already formed βA fibrils suggests that only the antigen binding site of the antibodies is involved in modulation of β-amyloid conformation.

### N-terminal EFRH sequence of β-amyloid peptide is the epitope of anti-aggregating antibodies

The existence of sequences that are kinetically involved in the folding process has previously been suggested in other systems and has been demonstrated by *in vitro* denaturation-renaturation experiments (Silen and Agard, 1989). Such sequences, which may play a role in the folding pathway, suggest the possibility that they serve not only for the folding process but may also contribute to conformational stability.

Identifying the "aggregating epitopes" as sequences that are related to the sites where protein aggregation is initiated, and preparing monoclonal antibodies against these regions, facilitates understanding and prevention of the protein aggregation processes.

The disaggregation as well as the prevention of amyloid was found to be dependent on the location of the epitopes on the β-amyloid and the binding characteristics of the mAbs (Solomon et al., 1997; Hanan and Solomon, 1996). The N-terminal region of the β-peptide was suggested to be the immunodominant site in AβP. Mabs raised against AβP fragments comprising amino acids 1-16 in the laboratory of the present inventors demonstrated that this region exhibits increased antigenic characteristics compared with the rest of the β-amyloid peptide.

Using the phage-peptide library, composed of filamentous phage displaying random combinatorial peptides, the EFRH residues (SEQ ID NO:5) located at positions 3-6 of the N-terminal AβP were defined as the epitope of anti-aggregating antibodies within βAP (Frenkel et al., 1998). The EFRH (SEQ ID NO:5) epitope is available for antibody binding when β-amyloid peptide is either in solution or is an aggregate, and locking of this epitope by antibodies affects the dynamics of all the molecules, preventing self-aggregation as well as enabling resolubilization of already formed aggregates. Identification of the epitope of mAb 2H3, which cannot affect β-amyloid formation despite the fact that it binds to the N-terminal of β-amyloid peptide, shed light on the importance of this specific sequence region, defined as an anti-aggregating epitope, on the behavior of the whole AβP molecule (Frenkel et al., 1999).

### Immunization against β-amyloid with EFRH phage as antigen

Locking of the EFRH (SEQ ID NO:5) epitope by site-directed antibodies was found to modulate the dynamics of aggregation as well as resolubilization of already formed aggregates. However, such small synthetic peptides, consisting of antibody epitopes, are generally poor antigens requiring the chemical synthesis of a peptide and need to be coupled to a large carrier, but even then they may induce a low affinity immune response. A novel immunization procedure for raising anti-AβP antibodies, using as antigen the filamentous phages displaying only EFRH (SEQ ID NO:5) peptide, was developed in the laboratory of the present inventors. Filamentous bacteriophages have been used extensively in recent years for the 'display' on their surface of large repertoires of peptides generated by cloning random oligonucleotides at the 5' end of the genes coding for the phage coat protein (Scott and Smith, 1990; Scott, 1992). As recently reported, filamentous bacteriophages are excellent vehicles for the expression and presentation of foreig peptides in a variety of biologicals (Greenwood et al., 1993; Medynski, 1994). Administration of filamentous phages induces a strong immunological response to the phage effects systems (Willis et al., 1993; Meola et al., 1995). Phage coat proteins pIII and pVIII are proteins that have been often used for phage display. The recombinant filamentous phage approach for obtaining specific peptide antigens has a major advantage over chemical synthesis, as the products obtained are the result of the biological fidelity of translational machinery and are not subject to the 70-94% purity levels common in the solid-phase synthesis of peptides. The phage presents an easily renewable source of antigen, as additional material can be obtained by growth of bacterial cultures.

Immunization with the EFRH (SEQ ID NO:5) displaying phage may, in a short period of time, raise the high concentration of high affinity (IgG) antibodies able to prevent the formation of β-amyloid and to minimize further toxic effects. The level of antibody in the sera was found to be related to the number of peptide copies per phage (Frenkel et al., 2000b).

The antibodies resulting from EFRH (SEQ ID NO:5) phage immunization are similar regarding their immunological properties to antibodies raised by direct injection with whole β-amyloid (Table 1). These antibodies recognize the full length β-peptide (1-40) and exhibit anti-aggregating properties as antibodies raised against whole Aβ peptide and/or β-amyloid (Frenkel et al., 2000b, 2001). The high immunogenicity of filamentous phages enables the raising of antibodies against self-antigens. Immunization of guinea pigs with EFRH (SEQ ID NO:5) phage as an antigen, in which the AβP sequence is identical to that in humans, resulted in the production of self-antibodies (Frenkel et al., 2001).

**Table 1: Competitive inhibition by various peptides within βAP of serum antibody raised against f88-EFRH compared to amyloid anti-aggregating antibody*.**

| **PEPTIDE** | **RESIDUES** | **MICE *SERUM*** | anti-aggregating antibody*. |
|---|---|---|---|
| FRH | (residues 4-6 of AβP) | ~10⁻³ M | 3 x10⁻³ M |
| EFRH | (residues 3-6 of AβP) and (SEQ ID NO:5) | 6.0x10⁻⁶ M | |
| | | | 3x10⁻⁶ M |
| DAEFRH | (residues 1-6 of AβP) and (residues 1-6 of SEQ ID NO:2) | 3.0x10⁻⁶ M | 8x10⁻⁷ M |
| DAEFRHD | (residues 1-7 of AβP) and (residues 1- 7 of SEQ ID NO:2) | 5.0x10⁻⁶ M | 9x10⁻⁷ M |
| DAEFRHDSG | (residues 1-9 of AβP) and (SEQ ID NO:2) | 5.0x10⁻⁶ M | 1x10⁻⁶ M |
| βAP(1-40) | | 3.0x10⁻⁶ M | 8x10⁻⁷ M |
| WVLD | (SEQ ID NO:3) | Nd ** | Nd ** |

| | | | |
|---|---|---|---|
| * Frenkel et. al. 1998 ** IC₅₀ value of less than 10⁻² M which cannot be detected by ELISA assay. | | | |

The above data demonstrated that a recombinant bacteriophage displaying a self-epitope can be used as a vaccine to induce autoantibodies for disease treatment. Filamentous phages are normally grown using a laboratory strain of *E*. *coli,* and although the naturally occurring strain may be different, it is reasonable to assume that delivery of phage into the gut will result in infection of the natural intestinal flora. The laboratory of the present inventors has found that UV inactivated phages are as immunogenic as their infective counterparts. There is evidence of long lasting filamentous phages in the guts of the immunized animals that may explain the long lasting immune response found in pill immunized mice (Zuercher et al., 2000).

Due to the high antigenicity of the phage, administration can be given by the intranasal route, which is the easiest way for immunization without any use of adjuvant. As olfactory changes are proposed to play a role in Alzheimer's disease (Murphy,1999) mucosal immunization is an effective induction of specific AβP IgA antibodies for preventing local pathologic effect of the disease.

The efficacy of phage-EFRH antigen in raising anti-aggregating β-amyloid antibodies (Solomon and Frenkel, 2000) versus whole β-amyloid shows that:
a. the high immunogenicity of the phage enables production of high titer of IgG antibodies in a short period of weeks without need of adjuvant administration;
b. self-expression of the antigen led to long-lasting immunization;
c. the key role of the EFRH epitope in β-amyloid formation and its high immunogenicity led to anti-aggregating antibodies which recognize whole β-amyloid peptide, substituting the use of β-amyloid fibrils.

### Performance of anti-β-amyloid antibodies in transgenic mice model of AD

Several laboratories have bred transgenic mice that produce Aβ and develop plaques and neuron damage in their brains (reviewed in Van Leuven, 2000). Although they do not develop the widespread neuron death and severe dementia seen in the human disease they are used as models for the study of Alzheimer's disease.

Production of anti-β-amyloid antibodies, by immunization with the fibrillar Aβ of the mouse model of AD, led to inhibition of the formation of amyloid plaques and the associated dystrophic neurites in the mouse brain (Schenk et al., 1999), and raises the feasibility of vaccination against AD. However, because of the low immunogenicity of the Aβ fibrils, repeated antigen administration in the presence of adjuvant is required to obtain the anti-AβP antibodies necessary to affect plaque formation. Moreover, immunizing with toxic fibrils may induce more accumulation of the toxic amyloid itself. Another set of experiments showed that peripheral administration of antibodies against amyloid β-peptide was sufficient to reduce amyloid burden in the affected mice brains (Bard et al., 2000). Despite their relatively modest serum levels, the passively administered antibodies were able to enter the central nervous system, decorate plaques and induce clearance of prexisting amyloid. Small amounts of such antibodies that cross the blood-brain barrier (0.1% of serum levels) might be sufficient to attenuate the further aggregation of these species into fibrillar Aβ dense-cored plaques. Because this pool of Aβ is small, and because antibodies to this form of Aβ might need only inhibit assembly of Aβ fibrils to have a functional effect, these antibodies need not necessarily cause large changes in total cerebral Aβ. These antibodies convert Aβ, the dense-cored plaques, to diffuse Aβ deposits.

By comparison, of the antibodies tested only mAbs 10D5, 3D6 and PabAβ_{1-42,} directed to the N-terminal regions of AβP demonstrated efficacy *in vivo.* In contrast, mAbs16C11, 21F12 and the control antibody TM2a, directed to other regions of AβP, were inactive. This result is consistent with the inability of these two antibodies to decorate plaques after in vivo administration and explains their inability to trigger plaque clearance (Bard et al., 2000). These in vivo data confirm the previous *in vitro* data (Solomon et al., 1996; 1997) that only antibodies directed to strategic epitopes, such as EFRH (SEQ ID NO:5), exhibit so-called 'chaperone-like' properties in dissolving the plaques and preventing their formation. These data also confirm that only a small amount of antibodies is necessary to interfere with non-covalent interactions between AβP fibrils to disaggregate them into an amorphous non-toxic configuration.

Appropriate animal models were used to test the effects of anti-β-amyloid antibodies on both brain damage and the cognitive losses caused by Alzheimer's disease. Indeed, immunization with β-amyloid peptide improves learning and memory, as well as diminishing brain damage in mouse models (Janus *et al.,* 2000; Morgan *et al.,* 2000; Chen et al., 2000). The results support a previously observed reduction in the formation of amyloid deposits but they go further to show that immunization also offered the mice some protection from the 'spatial' learning deficits that normally accompany plaque formation. Both groups suggest that either a small or selective reduction in β-amyloid deposition may be sufficient to protect against dementia. Each group used different tests of spatial memory, in which mice had to swim to and mount a platform located invisibly beneath the surface of a pool of water. It is remarkable that both groups find that immunization with β-amyloid peptide offers significant protection from the age- and amyloid-dependent performance deficits seen in non-immunized controls. Evidence of learning defects that depend on age (and are associated with increasing accumulation of β-amyloid peptide) was proved in one of the main mouse models of Alzheimer's disease (Chen *et al.* 2000). The authors show that these defects can be distinguished from age-independent deficits, but only by careful experimental design and analysis.

These findings indicate that Aβ overexpression and/or Aβ plaques are associated with disturbed cognitive function and, importantly, suggest that some but not all forms of learning and memory are suitable behavioral assays of the progressive cognitive deficits associated with Alzheimer's disease type pathologies.

The mechanism by which anti-β-amyloid antibodies blocks learning and memory deficits is not understood. One possibility is that the antibodies neutralize Aβ in some restricted compartment or deplete a non-deposited form of Aβ (for example, a soluble form) that is responsible for the memory loss observed (Morgan et al., 2000). Recently, soluble Aβ has been proposed as the cause of synapse loss in APP transgenic mice, as some transgenic lines develop reductions in synaptophysin immunoreactivity in dentate gyrus without developing Aβ deposits. A second possibility is that microglia activated by the antibodies can clear the deposited Aβ, thereby permitting normal cognitive function. An alternative explanation is that immunization affects Aβ in a particular conformation, like β-sheet forms in protofibrils. The former is more likely because of oligomeric assemblies of Aβ in β-sheets ('protofibrils') as an immunogen, and the resultant antisera preferentially recognized β-sheet forms of Aβ. This is significant because monoclonal antibodies raised to Aβ epitopes that initiate fibril aggregation inhibit assembly of synthetic Aβ oligomeric protofibrils *in vitro* (Solomon et al., 1996). It is possible, therefore, that the antibodies induced in the transgenic mice may bind to β-sheet oligomeric aggregates and inhibit further assembly. This Aβ species is especially neurotoxic, a critical intermediary in fibrillogenesis and an accurate predictor of neurodegeneration.

It is conceivable, however, that immunization might modulate Aβ metabolism through several distinct mechanisms, including destruction of Aβ by microglial phagocytosis (Schenk et al., 1999) or by effectory function of the antibody to activate Fc receptors able to remove the whole immunocomplex of fibrillar Aβ with site-directed antibodies.

US patent No. 5,229,490 describes an antigenic product comprising a denditric homopolymer containing monomeric residues, where a plurality of antigenic protein sequences are joined by covalent bonds (MAPS). This antigenic product, suitable for vaccines against various infectious diseases, provides an alternative carrier to natural carriers such as proteins, carbohydrates, lipids or liposomes.

However this document does not refer to the use of such products for the treatment of plaque-forming diseases such as Alzheimer's disease.

### SUMMARY OF THE INVENTION

The present invention provides an antigenic product that includes a multiple antigenic peptide (MAP) containing a plurality of an epitope of a deposit-forming polypeptide involved in a plaque-forming disease or disorder such as Alzheimer's disease. This MAP is based on a dendritic polymer built on a core molecule which is at least bifunctional/difunctional and containing up to 16 terminal functional groups to which an antigenic peptide that comprises an epitope of amyloid β comprising the amino acid sequence of SEQ ID NO:5 is joined by covalent bonds.

The present invention also provides an immunizing composition which contains the antigenic product according to of the present invention as an immunogen.

Another aspect of the present invention further provides a method for inducing an immune response against a deposit-forming polypeptide involved in a plaque-forming disease by administering the antigenic product of the present invention. The immune response induced is effective for prevention, inhibition of formation, and/or reabsorption of deposits of a plaque-forming disease, such as against Aβ in Alzheimer's Disease.

### BRIEF DESCRIPTION OF THE DRAWINGS

Figure 1 shows a schematic representation of an embodiment of a multiple antigenic peptide (MAP) on an octa-branched homo Wang resin. The arrow represents the cleavage site and the shaded YYEFRHDS (SEQ ID NO:1) is the antigenic peptide sequence.

Figure 2 shows a time line in weeks for injections and bleedings in the immunization and the monitoring of mice immunized with the MAP-YYEFRHDS (SEQ ID NO:1) antigenic product.

Figure 3 shows a graph of the titers of immunoglobulins at day 22 from a first bleeding after a second injection of MAP-YYEFRHDS (SEQ ID NO:1).

Figure 4 shows a graph of the titers of IgG and IgM in the sera of immunized mice by ELISA against the EFRH (SEQ ID NO:5) epitope at day 26 from a second bleeding after a second injection of MAP-YYEFRHDS (SEQ ID NO:1).

Figure 5 shows a graph of the IgG titers in the sera of immunized mice at day 70 from a second bleeding after a third injection of MAP-YYEFRHDS (SEQ ID NO:1).

Figure 6 shows a graph of the IgG titers in the sera of immunized mice at day 105 from a third bleeding after a third injection of MAP-YYEFRHDS (SEQ ID NO:1).

Figure 7 shows a comparison of the IgG titers in mouse sera 17 weeks after the third injection of MAP-YYEFRHDS (SEQ ID NO:1) .

Figure 8 shows a schematic representation of another embodiment of a multiple antigenic peptide on an octa-branched homo Wang resin. The arrow represents the cleavage site and the antigenic peptide sequence YEFRHYEFRH (SEQ ID NO:4) has a repeat of the EFRH (SEQ ID NO:5) Aβ epitope sequence. This MAP is also known as MAP-(EFRH)₂.

Figure 9 shows a time line in weeks for injections and bleedings in the immunization and monitoring of mice immunized with the MAP-YEFRHYEFRH (SEQ ID NO:4) antigenic product shown in Fig. 8 or the complex between streptavidin and biotinylated MAP shown in Fig. 11.

Figure 10 shows a graph of IgG titers in the serum of mice after immunization with MAP-YEFRHYEFRH (SEQ ID NO:4).

Figure 11 shows a schematic representation of a complex between streptavidin and biotinylated MAP.

Figure 12 shows a graph of IgG titers in the serum of mice after immunization with the complex between streptavidin and biotinylated MAP-YYEFRHDS (SEQ ID NO:1) as an embodiment of the complex shown in Fig. 11.

Figure 13 shows a schematic representation of still another embodiment of a multiple antigenic peptide on a tetra-branched homo Wang resin. Eight copies of the antigenic peptide sequence PrP (144-152) is present in MAP and is shown as DYEDRYYRE (SEQ ID NO:6).

Figure 14 shows a graph of the titers of IgG against the antigenic peptide sequence PrP (144-152) of SEQ ID NO:6. Two mice were immunized five times with MAP antigen having eight copies of SEQ ID NO:6 as the antigenic peptide sequence, which is part of helix 1 from PrP. The first immunization included complete Freund's adjuvant whereas the subsequent immunizations included incomplete Freund's adjuvant. Antibody level was measured using ELISA assay. Figures 13 and 14 are for illustrative purposes only.

### DETAILED DESCRIPTION OF THE INVENTION

The present invention is based on the discovery that a multiple antigen peptide carrying a multiplicity of the EFRH (SEQ ID NO:5) epitope of AβP had raised an unexpectedly superior immunological response compared with that obtained by immunization with either the full length AβP or the EFRH (SEQ ID NO:5) epitope displayed on the surface of a filamentous bacteriophage in the range of hundreds to thousands of copies per phage. In view of this unexpectedly superior immunological response to the EFRH (SEQ ID NO:5) epitopes in a multiple antigen peptide system, the present invention is intended to broadly encompass antigenic products carrying multiple copies of an epitope of a deposit-forming polypeptide involved in a plaque-forming disease in a multiple antigen peptide system.

As used herein in the specification and in the claims section that follows, the term "plaque-forming disease" refers to diseases characterized by formation of plaques by an aggregating protein (plaque forming peptide), such as, but not limited to, beta-amyloid, serum amyloid A, cystantin C, IgG kappa light chain, or prion protein (PrP), in diseases such as, but not limited to, early onset Alzheimer's disease, late onset Alzheimer's disease, presymptomatic Alzheimer's disease, SAA amyloidosis, hereditary Icelandic syndrome, senility, multiple myeloma, and to transmissible spongiform encephelopathy (TSE), also known as prion diseases that are known to affect humans, such as for example, kuru, Creutzfeldt-Jakob disease (CJD), Gerstmann-Straussler-Scheinker disease (GSS), and fatal familial insomnia (FFI) or animals, such as, for example, scrapie and bovine spongiform encephalitis (BSE).

The antigenic product according to the present invention is structurally based on a dendritic polymer in which antigens/epitopes are covalently bound to the branches that radiate from a core molecule. These dendritic polymers are characterized by higher concentrations of functional groups per unit of molecular volume than ordinary polymers. Generally, they are based upon two or more identical branches originating from a core molecule having at least two functional groups. Such polymers have been described by Denkewalter et al. in U.S. Patent No. 4,289,872 and by Tomalia et al. in several U.S. Patents including U.S. Patent Nos. 4,599,400 and 4,507,466. Other polymers of the class have been described by Erickson in U.S. Patent No. 4,515,920. The polymers are often referred to as dendritic polymers because their structure may be symbolized as a tree with a core trunk and several branches. Unlike a tree, however, the branches in dendritic polymers are all substantially identical. This dendrite system has been termed the multiple antigen peptide system (MAPS), which is the commonly used name for a combination antigen/antigen carrier that is composed of two or more, usually identical, antigenic molecules covalently attached to a dendritic core which is composed of principal units which are at least bifunctional/difunctional. Each bifunctional unit in a branch provides a base for added growth. The dendritic core of a multiple antigen peptide system can be composed of lysine molecules. For example, a lysine is attached via peptide bonds through each of its amino groups to two additional lysines. This second generation molecule has four free amino groups each of which can be covalently linked to an additional lysine to form a third generation molecule with eight free amino groups. A peptide may be attached to each of these free groups to form an octavalent multiple peptide antigen (MAP). The process can be repeated to form fourth or even higher generations of molecules. With each generation, the number of free amino groups increases geometrically and can be represented by 2ⁿ, where n is the number of the generation. Alternatively, the second generation molecule having four free amino groups can be used to form a tetravalent MAP, i.e., a MAP having four peptides covalently linked to the core. Many other molecules, including, e.g., aspartic acid and glutamic acid, both of which have two carboxyl groups and one amino group to produce polyaspartic or polyglutamic acids with 2ⁿ free carboxyl groups, can be used to form the dendritic core of a multiple antigen peptide system.

As will be apparent from the discussion hereinafter, some of the carrier or core molecules used to form the product of the present invention are of a molecular weight such that they might not usually be regarded as polymers. However, since their basic structure is similar to dendritic polymers, it is convenient to describe them as such. Therefore the term "dendritic polymer" will be sometimes used herein to define the product of the invention. The term includes carrier molecules which are sufficiently large to be regarded as polymers as well as those which may contain as few as three monomers.

The necessary chemistry for performing the synthesis of dendritic polymers is known and available. With amino acids, the chemistry for blocking functional groups which should not react and then removing the blocking groups when it is desired that the functional groups should react has been described in detail in numerous patents and articles in the technical literature. The dendritic polymers and the entire MAP can be produced on a resin as in Merrifield synthesis and then removed from the polymer. Tomalia utilized ammonia or ethylenediamine as the core molecule. In this procedure, the core molecule is reacted with an acrylate ester by Michael addition and the ester groups removed by hydrolysis. The resulting first generation molecules contain three free carboxyl groups in the case of ammonia and four free carboxyl groups when ethylenediamine is employed. Tomalia extends the dendritic polymer with ethylenediamine followed by another acrylic ester monomer, and repeats the sequence until the desired molecular weight is attained. It will, however, be readily apparent to one skilled in the art, that each branch of the dendritic polymer can be lengthened by any of a number of selected procedures. For example, each branch can be extended by multiple reactions with lysine molecules.

Erickson utilized the classic Merrifield technique in which a polypeptide of substantially any desired molecular weight is grown from a solid resin support. As the technique is utilized for the preparation of dendritic polymers, the linking molecule which joins the polymer to the resin support is trifunctional. One of the functional groups is involved in the linkage to the resin, the other two functional groups serve as the starting point for the growth of the polymer. The polymer is removed from the resin when the desired molecular weight has been obtained. One standard cleavage procedure is treatment with liquid hydrogen fluoride at 0°C. for one hour. Another, and more satisfactory procedure, is to utilize a complex of hydrogen fluoride and dimethylsulfide (HF:DMF) as described by Tam et al (1983). This procedure greatly minimizes side reactions and loss of peptide.

Denkewalter, in one example of his process, utilizes lysine as the core molecule. The amino groups of the core molecule are blocked by conversion to urethane groups. The carboxyl group is blocked by reaction with benzhydrylamine. Hydrolysis of the urethane groups generates a benzhydrylamide of lysine with two free amino groups which serve as the starting points for the growth of the dendritic polymer.
This brief outline of three of the available procedures for producing dendritic polymers should be adequate to teach those skilled in the art the basic principles of the current technology. They will also teach the skilled artisan the salient features of the polymers, one of the most important of which is that the polymers provide a large number of available functional groups in a small molecular volume. The result is that a high concentration of antigens in a small volume can be achieved by joining the antigen to those available functional groups. Moreover, the resulting molecular product contains a high proportion of antigen on a relatively small carrier, i.e., the ratio of antigen to carrier is quite high. This is in contrast to conventional products used as a basis for vaccines. These conventional products often are composed of a small amount of antigen on a large amount of carrier.

Other important features of the dendritic polymer as an antigen carrier are that the exact structure is known; there are no contaminants which may be themselves antigenic, produce tissue irritation or other undesirable reactions; the exact concentration of the antigen is known; the antigen is symmetrically distributed on the carrier; and the carrier can be utilized as a base for more than one antigen so that multivalent vaccines can be produced. The principal advantage of the MAPS of this invention as the basis for vaccines is that unlike previous systems using natural carriers such as keyhole limpet hemocyanin, tetanus toxoid and bovine serum albumin, the carriers of this invention are fully defined chemical entities on which the antigens are dispersed in known concentrations. Additionally, the antigen comprises a large part of the molecule, not a relatively small and undefined proportion of the molecule, as in the case of natural carriers.

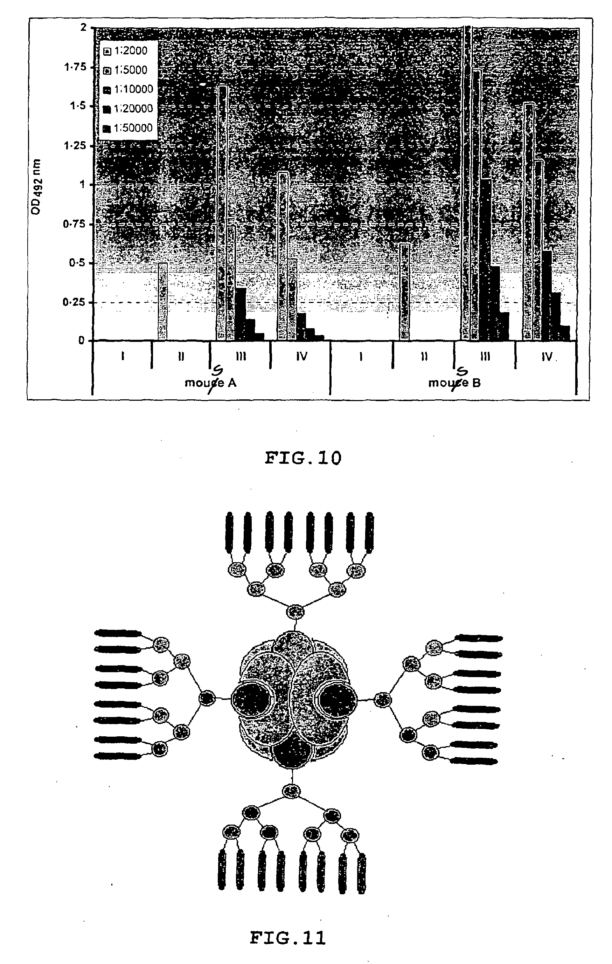

In addition to the dendritic polymer as a carrier, a streptavidin or avidin molecule can be used to further extend the size of the antigenic product and therefore avoid the use of adjuvants altogether for immunization and also to offer the ability to provide a plurality of MAP on a single molecule. Streptavidin and avidin are tetrameric proteins that carry one biotin binding site per monomer and therefore are capable of binding up to four biotin molecules. The multivalent feature of streptavidin or avidin can be used to form a complex with one to four biotinylated MAPs as a large antigenic product. The number of biotin sites bound with biotinylated MAP can be adjusted by varying the molar ratio of streptavidin (or avidin) :biotinylated MAP, such as 1:1, 1:2, 1:3, 1:4 or excess biotinylated MAP. This would allow one, two, three, or four biotinylated MAP to be bound in the complex with streptavidin or avidin. After binding of biotinylated MAP to streptavidin or avidin, any free biotin binding sites on streptavidin or avidin can be blocked with biotin. Fig. 11 shows a complex of streptavidin with four biotinylated octavalent MAP.

It may be that the number of epitopes of a deposit-forming polypeptide involved in a plaque-forming disease or disorder on the antigenic product of the present invention may affect how strong an immune response is generated by immunization with the antigenic product. This may explain why the presence of two epitopes per antigenic peptide on an octavalent MAP (for a total of 16 epitopes) in Example 2 raised a stronger immune reaction than the octavalent MAP in Example 1 (for a total of 8 epitopes). As the immune response elicited by the streptavidin:MAP complex of Example 3, which carries a total of about 32 epitopes per antigenic product of the present invention, appears to be lower than the immune response raised by the octavalent MAP of Example 2 with 16 total epitopes, the optimal number of epitopes per antigenic product may be in the range of greater than eight but less than 32. Although it has not yet been tested, the present inventor expects that a complex of streptavidin with four tetra-branched MAP having a total of 16 epitopes would be equivalent to a complex of streptavidin with two octa-branched MAP having a total of 16 epitopes provided that the epitopes in the tetra- and octa-branched MAP are the same.

As used herein, the term "biotin" or "biotinylated" is intended to encompass biotin, biocytin and other biotin analogs such as biotin amido caproate N-hydroxysuccinimide ester, biotin 4-amidobenzoic acid, biotinamide caproyl hydrazide and other biotin derivatives and conjugates. Other derivatives include biotin-dextran, biotin-disulfide-N-hydroxysuccinimide ester, biotin-6 amido quinoline, biotin hydrazide, d-biotin-N hydroxysuccinimide ester, biotin maleimide, d-biotin p-nitrophenyl ester, biotinylated nucleotides and biotinylated amino acids such as N-epsilon-biotinyl-1-lysine.

The terms "streptavidin" and "avidin" as used herein are meant to encompass native egg-white glycoprotein avidin and deglycosylated forms of avidin or stereptavidin, streptavidins produced by selected strains of *Streptomyces,* e.g., *Streptomyces avidinii,* either in their native 72 kDa or stable truncated 52-60 kDa forms, recombinant or chemically synthesized avidin or streptavidin, variants thereof with amino acid substitutions, derivatives thereof with chemical modifications, and fragments thereof as long as such "streptavidin" or "avidin" will still accommodate biotin binding. Some of these materials are commercially available, e.g., native avidin, degllycosylated avidins and streptavidin, or can be prepared by well-known methos (see Green 1975 for preparation of avidin and streptavidin; Bayer et al. 1995, for preparation of deglycosylated avidin). Recombinant avidin and recombinant streptavidin can be prepared by standard recombinant DNA techniques, for example, as described by Chandra and Gray, 1990, and by Argarana et al., 1986, for recombinant avidin and recombinant streptavidin, respectively. One avidin derivative, EXTRAVIDIN can be obtained in various functionally derivatized or conjugated forms from Sigma Chemical Company (St. Louis, MO). Another example of an avidin derivative is NEUTRALITE AVIDIN (Belovo Chemicals, Bastogne, Belgium), a deglycosylated form of avidin, which was obtained enzymatically, exhibits a neutral pI, and bears free lysine groups for further derivatization.

When the MAPS is to be employed to produce a vaccine, also referred to herein as an immunizing composition, it is preferred that the core molecule be a naturally occurring amino acid such as lysine so that it can be dealt with by the body following. the usual metabolic pathways. However, as will be explained more fully hereinafter, amino acids which are not naturally occurring, even those which are not α-amino acids can be employed. The acids, or any other asymmetric molecules used in building the core molecule can be in either the D or L form.

Although the dendritic polymers have been principally described hereinabove as polyamide polymers, it will be readily apparent that the carriers of this invention are not limited to dendritic polyamides. Any of a wide variety of molecules having at least two available functional groups can serve as core molecules. Propylene glycol, for example, can serve as the basis for a polyester dendritic polymer. Succinic acid with selected glycols or amines can serve as a core molecule to generate polyesters or polyamides. Diisocyanates can be used to generate polyurethanes. The important point is that the core molecule has at least two available functional groups from which identical branches can be generated by sequential scaffolding type reactions with additional molecules also having at least two available functional or anchoring groups on each branch. In the most simple case in which the core molecule has two available functional groups and each succeeding generation has two available functional groups, the number of anchoring sites to which antigen molecules can be anchored is expressed by 2ⁿ, where n is the number of the generation.

For a more complete discussion of the chemistry of dendritic polymers, attention is directed to Tomalia et al. (1985), Aharoni et al. (1982), and the following United States Pat. Nos. 4,289,872; 4,558,120; 4,376,861; 4,568,737; 4,507,466; 4,587,329; 4,515,920; 4,599,400; 4,517,122; and 4,600,535.

The present invention, in its presently preferred embodiments, provides a multiple antigen peptide system comprising a dendritic polymer base with a plurality of anchoring sites covalently bound to antigenic molecules which may be the same or different. The polymers comprise a central core molecule having at least two functional groups to which molecular branches having terminal functional groups are covalently bound. The terminal functional groups on the branches are covalently bonded to antigenic molecules, principally described herein as peptide antigens.

The selected antigen may be separately synthesized or otherwise obtained and joined to the carrier. Alternatively, the antigen may be synthesized on the carrier. For instance, if the antigen is an oligopeptide or relatively low molecular weight polypeptide, and the available functional groups on the polymer are amino groups or carboxyl groups, the antigen can be synthesized by extending each branch of the polymer utilizing known peptide synthesis techniques.

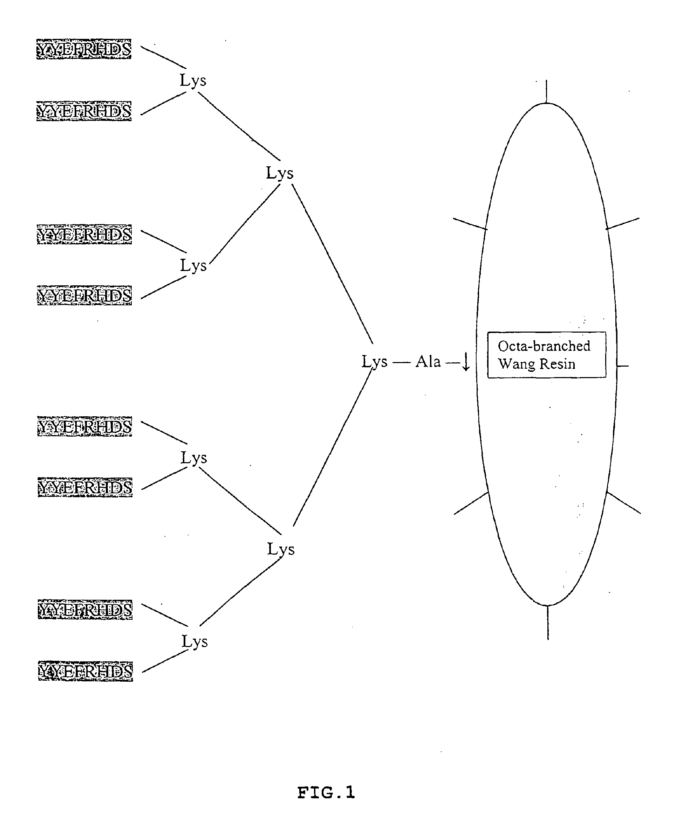

Fig. 1 shows the structure of a MAP dendritic polymer on a resin which may be employed in the practice of this invention. As will be seen, it is a three generation dendritic polylysine product. It may be obtained commercially, for example, as an octa-branched or tetra-branched Wang resin with a MAP core from a number of suppliers, i.e., Advanced ChemTech, Inc. Louisville, KY, or it may be produced by conventional solid phase techniques by generating the polymer on a Pam or a Pop resin. See Mitchell et al, (1978) and Tam et al, (1980). The polymer is then cleaved from the resin using, preferably HF:DMS. The dendritic polylysine, was built from a glycine linker originally joined through a benzyl linker to the resin. Other linkers such as alanine can be employed. Of course, the linker can be omitted, such as shown in Fig. 1, or a plurality of linker molecules can be utilized.

Fig. 1 shows a peptide antigen joined directly to each of the available functional groups on each terminal lysine moiety. In the case when the antigen is a relatively short peptide, e.g., 6 to 14 residues, it may be useful to extend the polylysine by a linker such as a simple tri- or tetrapeptide of glycine, alanine or beta alanine. However, for antigenic peptides with more than 14 residues, the linker is normally unnecessary.

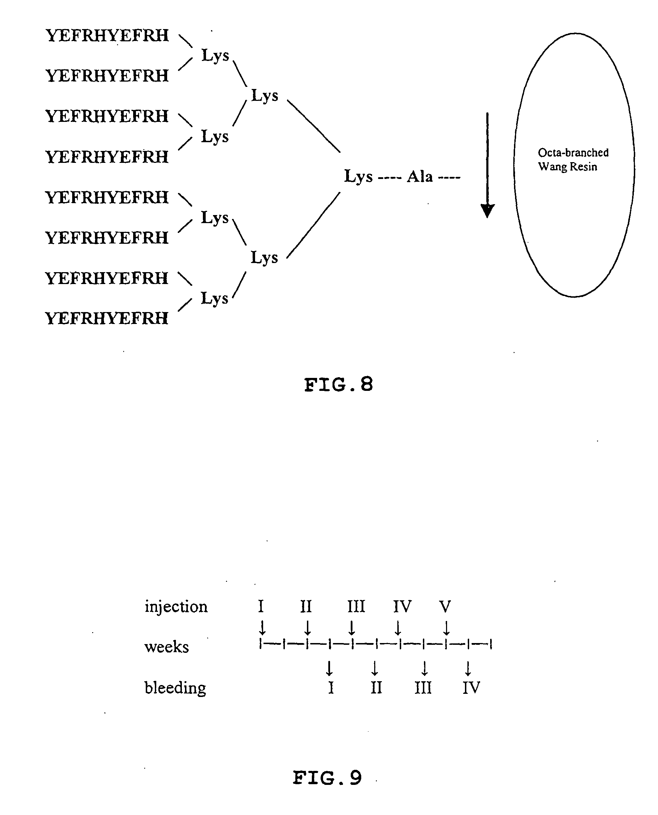

A preferred embodiment of an antigenic product, where a peptide antigen is joined to each of the available functional groups on each lysine moiety in an octavalent MAP, is shown in Fig. 8. This MAP-YEFRHYEFRH (SEQ ID NO:4) shown in Fig. 8 is also referred to herein as MAP-(EFRH)₂ because it has the EFRH (SEQ ID NO:5) epitope repeated twice in the peptide antigen. As demonstrated in Example 2, this antigenic product produced a strong immune response and is therefore a preferred embodiment of the present invention.

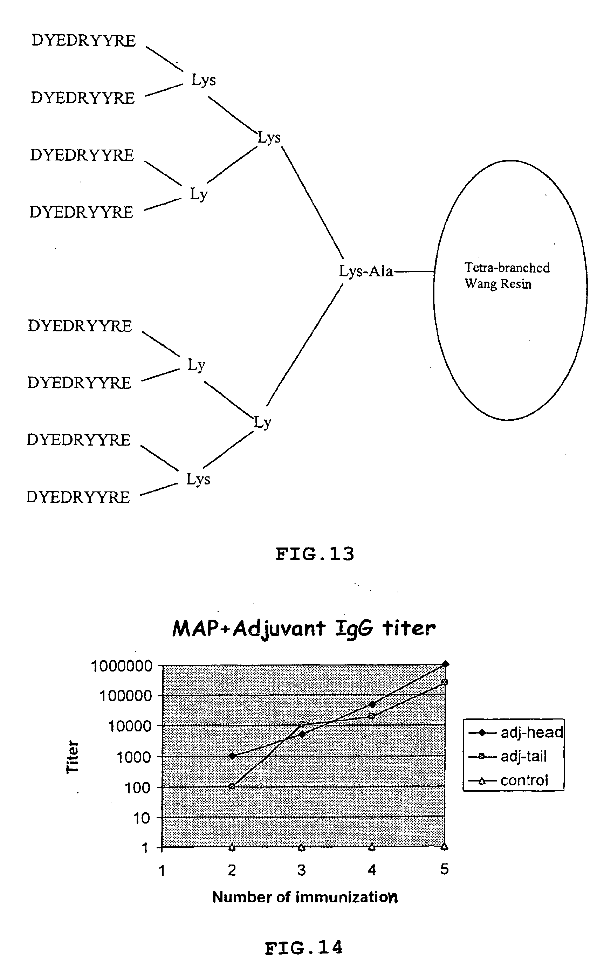

Further disclosed is an antigenic product where a peptide antigen PrP (144-152) (SEQ ID NO:6) is joined to each of the available functional groups on each lysine moiety in an octavalent MAP, is shown in Fig. 13. As demonstrated in Example 4, this antigenic product also produced a strong immune response and mentioned for illustrative purposes only.

The present invention has been described for convenience, principally as applied to products built on lysine as the core molecule. In fact lysine and lysine-like molecules such as ornithine, nor-lysine and amino alanine are preferred molecules for building the product of this invention because they are relatively easy to obtain, they are easy to work with, and they afford good yields. Such core molecules can be represented by the general formula:

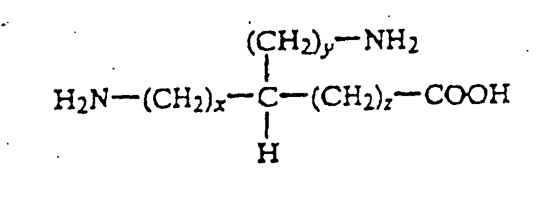

wherein x, y and z are integers from 0 to 10, preferably 0 to 4 provided that at least one of them is 1 and the amino groups cannot be attached to the same carbon atom. In the most preferred molecules, the total of x, y and z is from 2 to 6 and the amino groups are separated by at least two methylene groups.

Other preferred core molecules include ethylene diamine and like molecules with longer chains such as propylene diamine and butylene diamine. Such molecules may be represented by the general formula:

H₂N-CH₂- (CH₂) ₙ-CH₂-NH₂

wherein n is an integer from 0 to 10, preferably 0 to 3. Of course ammonia can also be employed as a core molecule.

The development of synthetic vaccines against a large number of diseases has been greatly accelerated because of the recognition that a vaccine need not be based on a native protein, but may be based on a low molecular weight segment of the native protein. These segments, normally called immunogenic determinants or epitopes are capable of stimulating the production of antibodies which will protect against, e.g., infection by an infectious vector of the native protein antigen. The immunogenic determinants are often low molecular weight peptides which can be conveniently synthesized. If they cannot be synthesized, they may be separated in pure form from the native protein itself.
Hereinafter, these antigenic immunostimulants will be referred to as antigenic peptides.

The principal embodiments of this invention may be broadly defined as antigenic products comprising a dendritic core molecule or polymer to which a plurality of antigens such as antigenic peptides containing epitopes of a deposit-forming polypeptide involved in a plaque deposit-forming disease or disorder are covalently bonded to the available functional groups. The antigens or epitopes may be different, although preferably the antigens or epitopes are the same.

More specifically, the principal embodiments of the invention may be defined as antigenic products or carrier systems comprising a dendritic polymer base which is a central core molecule having at least two available functional groups to which branches of selected lengths are joined. Each branch of the molecule terminates with at least one available, anchoring, functional group, a plurality of which are convalently bonded to antigenic molecules.

The antigenic peptide that is covalently joined to the available terminal functional groups on the dendritic polymer contains at least one copy of an epitope from a deposit-forming polypeptide, i.e., amyloid β, comprising the amino acid sequence of SEQ ID NO:5, involved in the formation of deposits in plaque-forming diseases or disorders. Non-limiting example of plaque-forming diseases or disorders include early onset Alzheimer's disease, late onset Alzheimer's disease, presymptomatic Alzheimer's disease, SAA amyloidosis, hereditary Icelandic syndrome, senility, multiple myeloma, kuru, Creutzfeldt-Jakob disease (CJD), Gerstmann-Straussler-Scheinker disease (GSS), and fatal familial insomnia (FFI). When more than one copy of an epitope, such as two or three copies is present on the antigenic peptide, a spacer of 1-8 amino acid residues, preferably 1-4 residues, separates the multiple copies of the epitope.

In the preferred embodiment of the present invention, the epitope is EFRH (SEQ ID NO:5). Non-limiting examples of an antigenic peptide containing this preferred epitope are shown in Table 1 and in Frenkel et al. (1998), with the antigenic peptide of SEQ ID NO:1 being most preferred. For small antigenic peptides, such as those having 6-12 residues, an octa-branched dendritic polymer (eight terminal functional groups) such as the octa-branched MAP Wang resin, is preferred. However, for larger peptides, in the range of about 20 amino acid residues or larger, a tetra-branched dendritic polymer, such as the tetra-branched MAP Wang resin is preferred. A preferred example of a larger peptide is an antigenic peptide that contains the residues 106-126 epitope of prion protein.

WO 00/72876 and WO 00/72880 disclose among a long list of possible examples, the use of MAP4 as a carrier for a peptide containing an Aβ epitope. There is no data in the publications showing that any of these constructs were actually made or tested. The unexpectedly superior immunogenicity of the MAP construct of the present invention is not disclosed or suggested by the above two publications. To the extent that any specific disclosure in these publications may be considered to anticipate any generic aspect of the present invention, the generic disclosure of the present invention should be understood as including a proviso excluding any such species previously disclosed in said publications. The aspects of the present invention which are not anticipated by the disclosure of said publications are unobvious from the disclosure of said publications because of the unexpectedly superior results disclosed or alleged herein.

An advantage of the present invention is that the dendritic polymer can serve as a carrier for two or more different antigens, if desired. One embodiment of this invention which utilizes this procedure is based on the use of a dendritic polylysine or other structurally similar molecule employing different amino blocking groups, one of which is stable to acid hydrolysis, the other of which is stable to alkaline hydrolysis. This makes it possible to protect either of the amino groups of lysine by the orthogonal protection method.

Fluorenylmethyloxycarbonyl (Fmoc) is a base labile protecting group and is completely stable to acidic deprotection. The t-butoxycarbonyl blocking group (Boc) is stable under basic conditions but not stable under mildly acidic conditions such as 50% trifluoroacetic acid. By choosing Boc-lys (Boc)-OH, Boc-lys (Fmoc)-OH, Fmoc-lys (Boc)-OH or Fmoc-lys (Fmoc)-OH, it is possible to place one set of antigens on the alpha amino group of lysine and another on the omega amino group. Those skilled in the art of peptide synthesis can readily devise methods of achieving the same types of products using diverse blocking groups and other dendritic polymers.

A few general observations applicable to the synthesis of MAPS will be of assistance to those skilled in the art. These are:

1. The syntheses generally require a long coupling time (2-4 hours).

2. Dimethyl formamide is generally a more suitable solvent than methylene dichloride.

3. The peptide resin should not be dried at any stage of the synthesis since resolvation is extremely difficult.

4. Coupling should be closely monitored for completion of the coupling by the quantitative ninhydrin method.

5. The MAPS is best cleaved from the resin by the improved acid deprotection method with either HF or TFMSA (Tam, et al., 1983 and 1986) in dimethyl sulfide to avoid strong acid catalyzed side reactions.

6. MAPS tend to strongly aggregate after cleavage from the resin support. Purification is best effected by extensive dialysis under basic and strongly denaturing conditions in a dialysis medium which is 8M in urea and mercaptoethanol to remove undesirable aromatic additives of the cleavage reactions such as p-cresol and thiocresol. Further purification, if desired, can be effected using high performance gel-permeation or ion exchange chromotography. In most cases the MAPS could be used directly without further purification.

It will be apparent to those skilled in the art that many variations of the structures shown and discussed herein are possible. All such variations are specifically included within the scope of this invention. For example, see U.S. Patent 5,229,490, the entire content of which is incorporated herein by reference.

The antigenic product of the present invention may include a lipophilic membrane anchoring moiety that confers adjuvant properties among its advantages. A lipophilic membrane-anchoring moiety at the carboxyl terminus of MAP enables further non-covalent amplification by a liposome or micellar form. Accordingly, the antigenic product according to the present invention may further be prepared with a variety of vehicles, including encapsulation within liposomes, for greater efficiency of delivery and concomitantly reduced dosage. The preparation of liposomes is well known in the art.

Tripalmitoyl-S-glyceryl cysteine (P3C) and palmitoyl lysine (PL) are non-limiting examples of suitable lipophilic moieties for the antigenic product of the present invention. P3C, which is a lipoamino acid from *Escherichia coli,* is a B cell mitogen that has proved particularly, successful as a non-toxic adjuvant. See U.S. Patent no. 5,580,563 and DeFoort et al., (1992), the entire contents of which are incorporated herein by reference.

Because the products of this invention provide a high concentration of antigen in a small molecular volume, in many instances the vaccines immunizing composition of the invention may be employed, without adjuvants. For instance, Example 3 demonstrates that a complex between streptavidin and MAP-YEFRHYEFRH (SEQ ID NO:4) elicited a strong immune response without an adjuvant. Generally, the larger the antigenic product of the present invention the less need there is for an adjuvant. However, if an adjuvant is employed, it may be selected from any of those normally employed to stimulate the immunogenic systems of mammals .

The vaccines/immunizing composition of the invention may be defined as comprising a pharmaceutically acceptable carrier together with an amount of an antigenic product of the invention which is sufficient to produce an immunological response. An effective amount may be very small. It will, as is known, vary with the antigen. With the product of this invention, because of the high concentration of antigen in a low molecular volume, it will be lower than with ordinary vaccines employing the same antigens. The quantity which constitutes an effective amount may vary depending on whether the vaccine is intended as a first treatment or as a booster treatment.

It may be convenient to provide the products of this invention as lyophilized or freeze dried powders ready to be reconstituted with a pharmaceutically acceptable carrier just prior to use.

The antigenic products of the present invention may also be employed in various diagnostic tests including radioimmunoassay, precipitation, complement fixation, direct and indirect immunofluorescence, agglutination and enzyme linked immunoassay. For such testing the diagnostic moiety joined to the dendritic polymer may be labeled with a detectable label, or it may be caused to react with a labeled product such as a labeled antibody to produce a detectable reaction product. Useful labels include fluorescent labels such as fluorescein, rhodamine or auramine. Radioisotopes such as ¹⁴C, ¹³¹I, ¹²⁵I and ³⁵S may be employed. Enzyme labels which may be utilized include, for example, horse radish peroxidase, β-D-glucosidase, β-D-galactosidase, urease, glucose oxidase plus peroxidase, and acid phosphatase. Methods for labeling are well known and need not be described.

In prophylactic applications, the antigenic product of the present invention is administered to a patient susceptible to, or otherwise at risk of, a plaque-forming disease such as Alzheimer's disease, in an amount sufficient to eliminate or reduce the risk, lessen the severity, or delay the onset of the disease, including biochemical, histologic and/or behavioral symptoms of the disease, its complications and intermediate pathological phenotypes presented during development of the disease. In therapeutic applications, the antigenic product of the invention is administered to a patient suspected of, or already suffering from such a disease in an amount sufficient to cure, or at least partially arrest, the symptoms of the disease (biochemical, histologic and/or behavioral), including its complications and intermediate pathological phenotypes in development of the disease. In some methods, administration of the antigenic product reduces or eliminates myocognitive impairment in patients that have not yet developed characteristic Alzheimer's pathology. An amount adequate to accomplish therapeutic or prophylactic treatment is defined as a therapeutically- or prophylactically- effective dosage. In both prophylactic and therapeutic regimes, the antigenic product of the present invention is usually administered in several doses until a sufficient immune response has been achieved. Typically, the immune response is monitored and repeated doses are given if the immune response starts to wane.

Effective dosages of the antigenic product of the present invention, for the treatment of a plaque-forming disease or disorder, such as Alzheimer's disease, vary depending upon many different factors, including means of administration, target site, physiological state of the patient, whether the patient is human or an animal, other medications administered, and whether treatment is prophylactic or therapeutic. Usually, the patient is a human but nonhuman mammals including transgenic mammals can also be treated. Treatment dosages need to be titrated to optimize safety and efficacy. The amount of immunogen depends on whether adjuvant is also administered, with higher dosages more likely to be required in the absence of adjuvant. The 1-500 µg per patient and more usually from 5-500 µg per injection for human administration. Occasionally, a higher dose of 1-2 mg per injection is used. Typically about 10, 20, 50 or 100 µg is used for each human injection. The mass of immunogen also depends on the mass ratio of immunogenic epitope within the immunogen to the mass of immunogen as a whole. The timing of injections can vary significantly from once a day, to once a year, to once a decade. On any given day that a dosage of immunogen is given, the dosage is greater than 1 µg/patient and usually greater than 10 µg/patient if adjuvant is also administered, and may be greater than 10 -100 µg/patient in the absence of adjuvant. A typical regimen consists of an immunization followed by booster injections at time intervals, such as 6 week intervals. Another regimen consists of an immunization followed by booster injections 1, 2 and 12 months later. Another regimen entails an injection every two months for life. Alternatively, booster injections can be on an irregular basis as indicated by monitoring of immune response.

The antigenic product of the present invention for inducing an immune response can be administered by parenteral, topical, intravenous, oral, subcutaneous, intraarterial, intracranial, intraperitoneal, intranasal or intramuscular means for prophylactic and/or therapeutic treatment. The most typical route of administration of an immunogenic agent is subcutaneous although other routes can be equally effective. The next most common route is intramuscular injection. This type of injection is mostly typically performed in the arm or leg muscles.

The antigenic product of the invention can sometimes be administered in combination with an adjuvant. A variety of adjuvants can be used in combination with the antigenic product of the invention to elicit an immune response. Preferred adjuvants augment the intrinsic response to an immunogen without causing conformational changes in the immunogen that affect the qualitative form of the response. Preferred adjuvants include aluminum hydroxide and aluminum phosphate, 3 De-O-acylated monophosphoryl lipid A (MPL^{™}) (see GB 2220211, RIBI ImmunoChem Research Inc., Hamilton, Montana, now part of Corixa). Stimulon^{™} QS-21 is a triterpene glycoside or saponin isolated from the bark of the Quillaja Saponaria Molina tree found in South America (see Kensil et al., 1995); US Patent No. 5,057,540 (Aquila BioPharmaceuticals, Framingham, MA). Other adjuvants are oil in water emulsions (such as squalene or peanut oil), optionally in combination with immune stimulants, such as monophosphoryl lipid A (see Stoute et al., 1997). Another adjuvant is CpG (WO 98/40100). Alternatively, the antigenic product can be coupled to an adjuvant. However, such coupling should not substantially change the conformation of the epitope so as to affect the nature of the immune response thereto. Adjuvants can be administered as a component of a composition with the antigenic product of the invention administered separately, before, concurrently with, or after administration of antigenic product.

A preferred class of adjuvants is aluminum salts (alum), such as aluminum hydroxide, aluminum phosphate, aluminum sulfate. Such adjuvants can be used with or without other specific immunostimulating agents such as MPL or 3-DMP, QS-21, polymeric or monomeric amino acids such as polyglutamic acid or polylysine. Another class of adjuvants is oil-in-water emulsion formulations. Such adjuvants can be used with or without other specific immunostimulating agents such as muramyl peptides (e.g., N-acetylmuramyl-L-threonyl-D-isoglutamine (thr-MDP), N-acetyl-normuramyl-L-alanyl-D-isoglutamine (nor-MDP), N-acetylmuramyl-L-alanyl-D-isoglutarninyl-L-alanine-2-(1'-2'dipalmitoyl-sn-glycero-3-hydroxyphosphoryloxy)-ethylamine (MTP-PE), N-acetylglucsaminyl-N-acetylmuramyl-L-Al-D-isoglu-L-Ala-dipalmitoxy propylamide (DTP-DPP) theramide^{™}), or other bacterial cell wall components. Oil-in-water emulsions include (a) MF59 (WO 90/14837), containing 5% Squalene, 0.5% Tween 80, and 0.5% Span 85 (optionally containing various amounts of MTP-PE) formulated into submicron particles using a microfluidizer such as Model 110Y microfluidizer (Microfluidics, Newton MA), (b) SAF, containing 10% Squalene, 0.4% Tween 80, 5% pluronic-blocked polymer L121, and thr-MDP, either microfluidized into a submicron emulsion or vortexed to generate a larger particle size emulsion, and (c) Ribi^{™} adjuvant system (RAS) (Ribi ImmunoChem, Hamilton, MT) containing 2% squalene, 0.2% Tween 80, and one or more bacterial cell wall components from the group consisting of 5 monophosphoryllipid A (MPL), tiehalose dimycolate (TDM), and cell wall skeleton (CWS), preferably MPL + CWS (Detox^{™}). Another class of preferred adjuvants is saponin adjuvants, such as Stimulon^{™} (QS-21, Aquila, Framingham, MA) or particles generated therefrom such as ISCOMs (immunostimulating complexes) and ISCOMATRIX. Other adjuvants include Complete Freunds Adjuvant (CFA) and Incomplete Freunds Adjuvant (IFA). Other adjuvants include cytokines, such as interleukins (IL-1, IL-2, and IL-12), macrophage colony stimulating factor (M-CSF), tumor necrosis factor (TNF).

An adjuvant can be administered with an immunogen as a single composition, or can be administered before, concurrent with or after administration of the immunogen. Immunogen and adjuvant can be packaged and supplied in the same vial or can be packaged in separate vials and mixed before use. Immunogen and adjuvant are typically packaged with a label indicating the intended therapeutic application. If immunogen and adjuvant are packaged separately, the packaging typically includes instructions for mixing before use. The choice of an adjuvant and/or carrier depends on the stability of the immunogenic formulation containing the adjuvant, the route of administration, the dosing schedule, the efficacy of the adjuvant for the species being vaccinated, and, in humans, a pharmaceutically acceptable adjuvant is one that has been approved or is approvable for human administration by pertinent regulatory bodies. For example, Complete Freund's adjuvant is not suitable for human administration. Alum, MPL and QS-21 are preferred. Optionally, two or more different adjuvants can be used simultaneously. Preferred combinations include alum with MPL, alum with QS-21, MPL with QS-21, and alum, QS-21 and MPL together. Also, Incomplete Freund's adjuvant can be used (Chang et al., 1998), optionally in combination with any of in QS-2, and WPL and all combinations thereof.

The antigenic product of the present invention is often administered as pharmaceutical compositions comprising an active agent, i.e., the antigenic product, and a variety of other pharmaceutically acceptable components. See Remington's Pharmaceutical Science (15^{th} ed., Mack Publishing Company, Easton, Pennsylvania, 1980). The preferred form depends on the intended mode of administration and therapeutic application. The compositions can also include, depending on the formulation desired, pharmaceutically acceptable, non-toxic carriers or diluents, which are defined as vehicles commonly used to formulate pharmaceutical compositions for animal or human administration. The diluent is selected so as not to affect the biological activity of the combination. Examples of such diluents are distilled water, physiological phosphate-buffered saline, Ringer's solutions, dextrose solution, and Hank's solution. In addition, the pharmaceutical composition or formulation may also include other carriers, adjuvants, or auxiliary agents or nontoxic, nontherapeutic, nonimmunogenic stabilizers and the like.

Pharmaceutical compositions can also include large, slowly metabolized macromolecules such as proteins, polysaccharides such as chitosan, polylactic acids, polyglycolic acids and copolymers (such as latex functionalized sepharose^{™}, agarose, cellulose, and the like), polymeric amino acids, amino acid copolymers, and lipid aggregates (such as oil droplets or liposomes). Additionally, these carriers can function as immunostimulating agents (i.e., adjuvants).

For parenteral administration, the antigenic product of the present invention can be administered as injectable dosages of a solution or suspension of the substance in a physiologically acceptable diluent with a pharmaceutical carrier that can be a sterile liquid such as water oils, saline, glycerol, or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above (see Langer, 1990 and Hanes, 1997).

Patients amenable to treatment include individuals at risk of a plaque-forming disease but not showing symptoms, as well as patients presently showing symptoms. In the case of Alzheimer's disease, virtually anyone is at risk of suffering from Alzheimer's disease if he or she lives long enough. Therefore, the present antigenic product can be administered prophylactically to the general population without the need for any assessment of the risk of the subject patient. The present methods are especially useful for individuals who do have a known genetic risk of Alzheimer's disease. Such individuals include those having relatives who have experienced this disease, and those whose risk is determined by analysis of genetic or biochemical markers. Genetic markers of risk toward Alzheimer's disease include mutations in the APP gene, particularly mutations at position 717 and positions 670 and 671 referred to as the Hardy and Swedish mutations respectively. Other markers of risk are mutations in the presenilin genes, PS1 and PS2, and ApoE4, family history of AD, hypercholesterolemia or atherosclerosis. Individuals presently suffering from Alzheimer's disease can be recognized from characteristic dementia, as well as the presence of risk factors described above. In addition, a number of diagnostic tests are available for identifying individuals who have AD. These include measurement of CSF tau and Aβ42 levels. Elevated tau and decreased Aβ42 levels signify the presence of AD. Individuals suffering from Alzheimer's disease can also be diagnosed by ADRDA criteria.

In asymptomatic patients, treatment can begin at any age (e.g., 10, 20, 30). Usually, however, it is not necessary to begin treatment until a patient reaches 40, 50, 60 or 70. Treatment typically entails multiple doses over a period of time. Treatment can be monitored by assaying antibody, or B-cell responses to the therapeutic agent (e.g., antigenic product of the present invention) over time. If the response falls, a booster dose is indicated. In the case of potential Down's syndrome patients, treatment can begin antenatally by administering therapeutic agent to the mother or shortly after birth.

The present invention also provides for methods of detecting an immune response against a deposit-forming polypeptide involved in a plaque deposit-forming disease in a patient suffering from or susceptible to, e.g., Alzheimer's disease. The methods are particularly useful for monitoring a course of treatment being administered to a patient. The methods can be used to monitor both therapeutic treatment on symptomatic patients and prophylactic treatment on asymptomatic patients by monitoring antibody produced in response to administration of immunogen.

Some methods entail determining a baseline value of an immune response in a patient before administering a dosage of the antigenic product, and comparing this with a value for the immune response after treatment. A significant increase (i.e., greater than the typical margin of experimental error in repeat measurements of the same sample, expressed as one standard deviation from the mean of such measurements) in value of the immune response signals a positive treatment outcome (i.e., that administration of the agent has achieved or augmented an immune response). If the value for immune response does not change significantly, or decreases, a negative treatment outcome is indicated. In general, patients undergoing an initial course of treatment with an immunogenic agent are expected to show an increase in immune response with successive doses, which eventually reaches a plateau. Administration of agent is generally continued while the immune response is increasing. Attainment of the plateau is an indicator that the administered of treatment can be discontinued or reduced in dosage or frequency.

In other methods, a control value (i.e., a mean and standard deviation) of immune response is determined for a control population. Typically the individuals in the control population have not received prior treatment. Measured values of immune response in a patient after administering a therapeutic agent are then compared with the control value. A significant increase relative to the control value (e.g., greater than one standard deviation from the mean) signals a positive treatment outcome. A lack of significant increase or a decrease signals a negative treatment outcome. Administration of agent is generally continued while the immune response is increasing relative to the control value. As before, attainment of a plateau relative to control values is an indicator that the administration of treatment can be discontinued or reduced in dosage or frequency.

In other methods, a control value of immune response (e.g., a mean and standard deviation) is determined from a control population of individuals who have undergone treatment with a therapeutic agent and whose immune responses have plateaued in response to treatment. Measured values of immune response in a patient are compared with the control value. If the measured level in a patient is not significantly different (e.g., more than one standard deviation) from the control value, treatment can be discontinued. If the level in a patient is significantly below the control value, continued administration of agent is warranted. If the level in the patient persists below the control value, then a change in treatment regime, for example, use of a different adjuvant may be indicated.

In other methods, a patient who is not presently receiving treatment but has undergone a previous course of treatment is monitored for immune response to determine whether a resumption of treatment is required. The measured value of immune response in the patient can be compared with a value of immune response previously achieved in the patient after a previous course of treatment. A significant decrease relative to the previous measurement (i.e., greater than a typical margin of error in repeat measurements of the same sample) is an indication that treatment can be resumed.

Alternatively, the value measured in a patient can be compared with a control value (mean plus standard deviation) determined in a population of patients after undergoing a course of treatment. Alternatively, the measured value in a patient can be compared with a control value in populations of prophylactically treated patients who remain free of symptoms of disease, or populations of therapeutically treated patients who show amelioration of disease characteristics. In all of these cases, a significant decrease relative to the control level (i.e., more than a standard deviation) is an indicator that treatment should be resumed in a patient.

The tissue sample for analysis is typically blood, plasma, serum, mucous or cerebrospinal fluid from the patient. The sample is analyzed for indication of an immune response to the antigenic product. The immune response can be determined from the presence of, e.g., antibodies that specifically bind to Aβ peptide. ELISA methods of detecting antibodies specific to Aβ peptide are described in the Examples below.

Having now generally described the invention, the same will be more readily understood through reference to the following example which is provided by way of illustration and is not intended to be limiting of the present invention.

### EXAMPLE 1

### Preparation of MAP with the EFRH (SEQ ID NO:5) epitope

The peptide chain TyrTyrGluPheArgHisAspSer (SEQ ID NO:1) was synthesized (growing from C- to N-temini) on an octa-branched Wang Resin, resin-beta Ala-Lys-2Lys-4Lys-4Fmoc, which is a MAP core resin. The octa-branched Wang resin was obtained from the supplier, Advanced ChemTech, Inc., Louisville, KY (www.peptide.com) and has a cleavable part consisting of beta-alanine to which seven lysines, branched like a tree, are attached. The branches terminate at four lysines with two Fmoc groups each for a total of eight Fmoc groups. The synthesis of MAP can be performed according to the supplier's instructions or according to any number of peptide synthesis protocols such as disclosed in U.S. Patent 5,229,490, and Tam et al. (1989).

### Preparation of the antigen

The stock of MAP-YYEFRHDS (SEQ ID NO:1; 2 mg/ml) was prepared in 12.5% DMF. 2 mg of MAP-YYEFRHDS (SEQ ID NO:1) was dissolved in 125 µl of DMF and 875 µl of double distilled water was then added to bring the volume up to 1 ml. The stock was stored in a freezer.

### Immunization of Balb/C mice

Four mice (named A, B, C, D) were immunized at two week intervals with 100 µg of MAP-YYEFRHDS (SEQ ID NO:1) + adjuvant (either Complete Freund's or Incomplete Freund's adjuvant) by intraperitoneal injection, and two mice were immunized with H₂O + adjuvant as a negative control. All mice were 8 week old female mice.

### Immunization protocol

Day 0 - the first injection
Day 15 - the second injection
Day 28 - the third injection

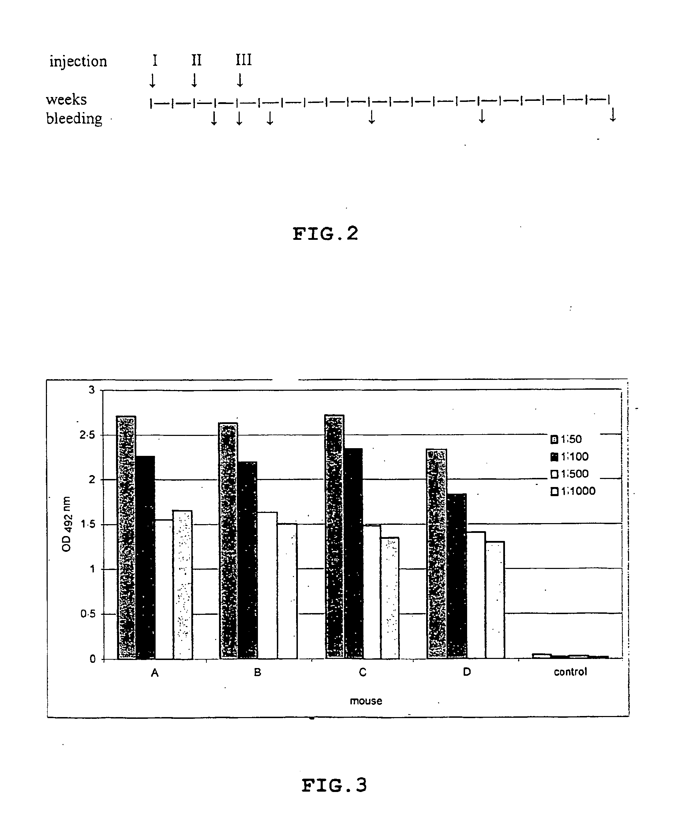

The time line (in weeks) for injections and bleedings is shown in Fig. 2.

### Binding of polyclonal antibodies to β-amyloid peptide

In experiments of antibody binding to β-amyloid peptide 1-16, 100 ng/well biotinylated β-amyloid peptides 1-16 were bound to streptavidin-coated microtiter plates for ½ h at room temperature. ELISA plates were previously coated with streptavidin for 16 h at 4°C. after washing, bound antibodies to β-peptide were detected by incubation with horseradish peroxidase-conjugated antibodies, as previously described in Frenkel et al. (1999). The Ig type was measured with rabit anti-mouse IgG and anti-mouse IgM labeled with horseradish peroxidase.

### Analysis of immunological response

As can be seen from the results after ELISA against the EFRH-epitope at various dilutions (Fig. 3), the immune response is similar for each mouse.

### Determination of Ig type in sera of immunized mice with ELISA against EFRH-epitope (SEQ ID NO:5)

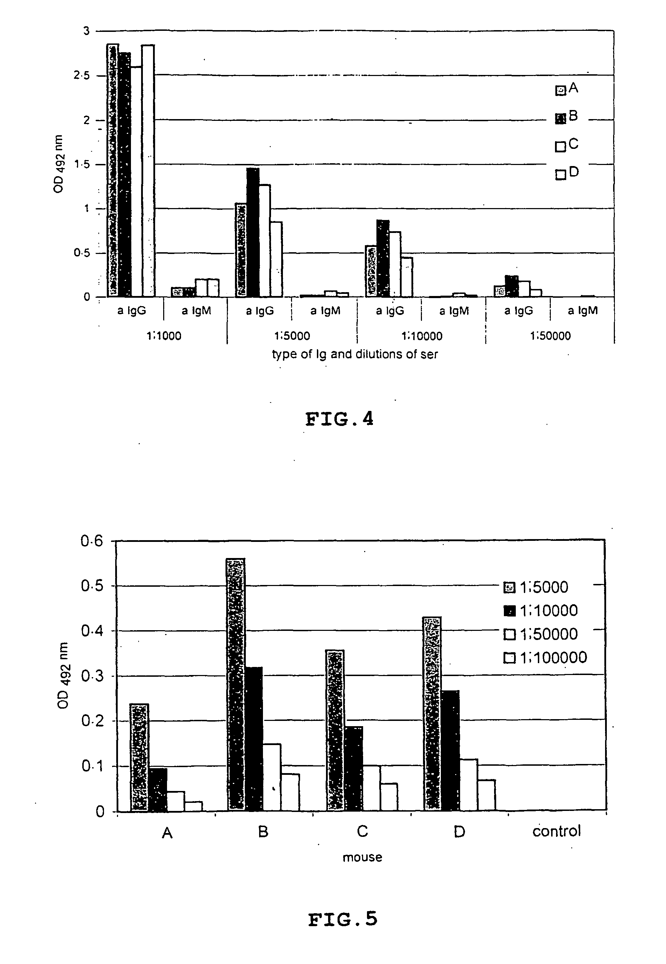

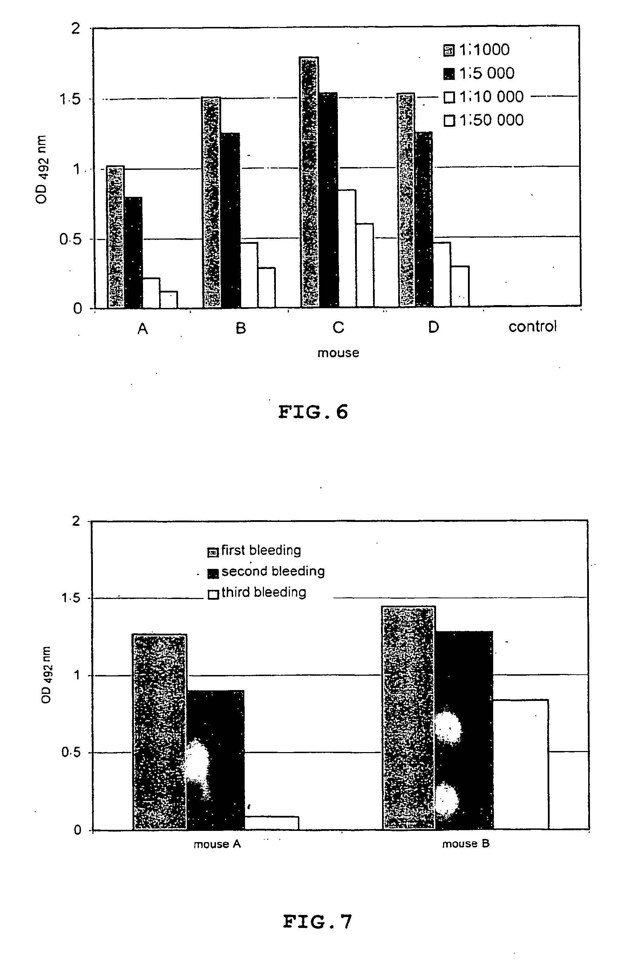

Fig. 4 shows that the overwhelming majority of antibodies is IgG and therefore provides evidence of a strong second immune response. As seen from results of Fig. 5, each mouse achieved an adequate immune response, but mouse B showed a higher titer. At day 105, the IgG titer in the sera of immunized mice is still quite high (Fig. 6).

### Long lasting presence of IgG against EFRH-epitope (residues 3-6 of SEQ ID NO:1)

As can be seen from Fig. 7, the immune response is still strong after 17 weeks from the third injection of MAP-YYEFRHDS (SEQ ID NO:1), which is 21 weeks after the first injection of MAP-YYEFRHDS (SEQ ID NO:1).

### EXAMPLE 2

The peptide chain TyrGluPheArgHisTyrGluPheArgHis (SEQ ID NO:4), which has two EFRH (SEQ ID NO:5) epitopes and is also known as MAP-(EFRH)₂, was synthesized in the same manner as the peptide chain in Example 1. The resulting MAP-(EFRH)₂ is shown in Fig. 8. The stock of the antigen MAP-(EFRH)₂ (10 mg/ml) was prepared in 10% DMF and was stored in a freezer.

### Immunization of Balb/c mice

Two female mice 8 weeks old (named A and B) were immunized at approximately two week intervals with 100µg of MAP-(EFRH)₂ + filamentous phage as adjuvant by intraperitoneal injection.

### Immunization protocol

Day 0 - the first injection of 3x10² phage + MA-(EFRH)₂ 100 µg per mouse.
Day 15 - the second injection 3x10² phage + MAP-(EFRH)₂ 100 µg per mouse.
Day 34 - the third injection 3x10² phage + MAP-(EFRH)₂ 100 µg per mouse.
Day 48 - the fourth injection MAP-(EFRH)₂ (100 µg) without phage.
Day 62 - the fifth injection MAP-(EFRH)₂ (100 µg).

The approximate time line (in weeks) for injections and bleedings is shown in Fig. 9.

### Immunological response

As can be seen from the results after ELISA against the EFRH-epitope at various dilutions (Fig. 10), immunization with MAP-(EFRH)₂ produced a strong immune response as demonstrated by the high serum IgG titers.

### EXAMPLE 3

The MAP-YYEFRHDS (SEQ ID NO:1) antigen prepared in Example 1 was biotinylated, and the biotinylated antigen was added to steptavidin in four portions in tubes during each 30 min. The tubes were incubated at room temperature with gentle shaking. The steptavidin:MAP-YYEFRHDS (SEQ ID NO:1) molar ratio was 1:4. After incubation, the mix was dialyzed against PBS or H₂O. The resulting complex of streptavidin and biotinylated MAP-YYEFRHDS (SEQ ID NO:1) is shown in Fig. 11.

### Immunization of Balb/c mice

Two female mice 8 weeks old (named G and H) were immunized at approximately two week intervals with the streptavidin and biotinylated MAP-YYEFRHDS (SEQ ID NO:1) complex (100 µg of MAP-YYEFRHDS (SEQ ID NO:1) per mouse) by intraperitoneal injection without adjuvant.

### Immunization protocol

Day 0 - the first injection
Day 15 - the second injection
Day 34 - the third injection
Day 48 - the fourth injection
Day 62 - the fifth injection

The approximate time line (in weeks) for injections and bleedings is shown in Fig. 9.

### Immunological response

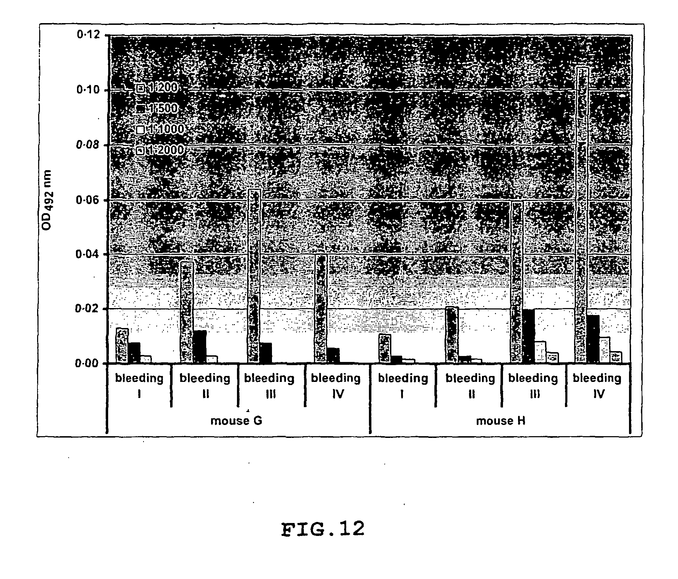

As can be seen from the results after ELISA against the EFRH-epitope at various dilutions (Fig. 12), immunization with the complex of streptavidin and biotinylated MAP-YYEFRHDS (SEQ ID NO:1) produced a good immune response, although not as strong as the immune response observed in Example 2 when serum IgG titers are compared.

### EXAMPLE 4

### (for illustrative purpose only)

The antigenic peptide is human PrP 144-152, DYEDRYYRE (SEQ ID NO:6), the sequence recognized by monoclonal antibody 6H4 (Prionics AG), with the designed MAP presenting eight copies of this antigenic peptide (Fig. 13).

The animals used as a research model for active immunization were eight week old female BALB/C mice, where the respective sequence in mice is different from that of humans, i.e., the mouse sequence is 143-151 DWEDRYYRE (SEQ ID NO:7), which has one deletion and one mismatch, Tyr to Trp, when compared to the human PrP 144-152 sequence.

Mice were immunized once every two weeks with 100 µg of MAP PrP (144-152) associated with Freund's adjuvant and bled every other week in order to examine the antibody titer. The first immunization was performed with CFA (complete Freund's adjuvant) while the other four were carried out with IFA (incomplete Freund's adjuvant).

The mice were bled from the eye vein and the antibody titer against the peptide was measured using ELISA assay. IgG antibodies raised against the peptide were observed after the second immunization and reached their peak after the fifth immunization, with a titer of 1:1,000,000 (Fig. 14). As expected during that period of time, the IgM antibody level dropped to a titer of 1:50. Immunization was ceased after the fifth immunization, and eight weeks later the mice were bled again and antibody level was found to reach a titer of 1:100,000. Moreover, the sera recognized the whole PrP protein by immunostaining CHO cells.

### REFERNCES

Argarana et al., *Nucleic Acid Res.,* 14:1871-1882 (1986)
Bard, F., Cannon, C., Barbour, R., Burke, R-L., Games, D., Grajeda, H., Guido, T., Hu, K., Huang, J., Johnson-Wood, K., Khan, K., Kholodenko, D., Lee, M., Lieberburg, I., Motter, R., Nguyen, M., Soriano, F., Vasquez, N., Weiss, K., Welch, B., Seubert, P., Schenk, D. and Yednock, T. Peripherally administered antibodies against amyloid beta peptide enter the central nervous system and reduce pathoogy in a mouse model of Alzheimer disease. *Nature Medicine,* 6:916-920 (2000).
Barrow, C.J. and Zagorski, M.G. Solution structures of β-peptide and its constituent fragments: Relation to amyloid deposition. *Science* 253, 179-182 (1991).
Bayer et al., *App. Biochem. Biotech.,* 53:1-9 (1995)
Blond, S. and Goldberg, M. Partly native epitopes are already present on early intermediates in the folding of tryptophan synthase. *Proc Natl Acad Sci USA* 84, 1147-1151 (1987).
Carlson, J.D. and Yarmush, M.L. Antibody assisted protein refolding. *Biotechnol.* 10, 86-91 (1992).
Carrell, R.W., Lomas, D.A. Conformational disease. *Lancet* 350, 134-138 (1997).
Chandra et al., *Meth. Enzymol.,* 184:70-79 (1990)
Chang et al., *Advanced Drug Delivery Reviews,* 32:173-186 (1998)
Chen, G., Chen, K.S., Knox, J., Inglis, J., Bernard, A., Martin, S.J., Justice, A., McConlogue, L., Games, D., Freedman, S.T. and Morris, R.G.M. A learning deficit related to age and β-amyloid plaques in a mouse model of Alzheimer's disease. *Nature* 408:975-979 (2000).
DeFoort et al., Int. J. Peptide Protein Res., 40:214-221 (1992)
Fraser, P. E., Levesque, L. and McLachlan, D.A. Biochemistry of Alzheimer's disease, amyloid plaques. *Clin. Biochem. 26,* 339-349 (1993).
Frenkel, D., Balass, M. and Solomon, B. N-Terminal EFRH sequence of Alzheimer's β-amyloid peptide represents the epitope of its anti-aggregating antibodies. J *Neuroimmunology,* 88*,* 85-90 (1998).
Frenkel, D., Balass, M., Kachalsky-Katzir, E. and Solomon, B. High affinity binding of monoclonal antibodies to the sequential epitope EFRH of β-amyloid peptide is essential for modulation of fibrillar aggregation. J. *Neuroimmunology* 95, 136-142 (1999).
Frenkel, D., Solomon, B. and Benhar, I. Modulation of Alzheimer's beta-amyloid neurotoxicity by site-directed single-chain antibody. *J Neuroimmunol,* 106:23-31 (2000a).
Frenkel, D., Katz, O. and Solomon, B. Immunization against Alzheimer's β-amyloid plaques via EFRH phage administration. *PNAS* 97, 21, 11455-11459 (2000b).
Frenkel, D., Kariv, N. and Solomon, B. Generation of autoantibodies towards Alzheimer's disease vaccination. Vaccine 19, 2615-2619 (2001).
Frauenfelder, H., Petsko, G.A. and Tsernoglou, D. Temperature dependent X-ray diffraction as a probe of protein structural dynamics. *Nature,* 280, 558-563 (1979).
Goate, A., Chartier-Harlin, M-C., Mullan, M. et al. (1991). Segregation of a missense mutation in the amyloid precursor protein gene with familial Alzheimer's disease. *Nature* 349, 704-706 (1991).
Gray, *Arch., Biochem. Biophys.,* 163:426-428 (1974)
Green, *Advances in Protein Chemistry,* 29:85-133 (1975)
Greenwood, J., Willis, E.A. and Perham, N.R. Multiple display of foreign peptides on a filamentous bacteriophage. *J Mol Biol,* 220, 821-827 (1993).
Hanan, E. and Solomon, B. Inhibitory effect of monoclonal antibodies on Alzheimer's β-amyloid peptide aggregation. Amyloid: *Int. J. Exp. Clin. Invest.* 3, 130-133 (1996).
Hanes, *Advanced Drug Delivery Reviews,* 28:97-119 (1997)
Hardy, J. Amyloid, the presenilins and Alzheimer's disease. *Trends Neurosci.* 20, 154-159 (1997).
Hardy, J. and Allsop, D. Amyloid deposition as the central event in the aetiology of Alzheimer's disease. *Trends Pharmacol Sci.* 12, 18295-18298 (1991).
Hardy, J., Duff, K., Hardy, K.G., Perez-Tur, J. and Hutton, M. Genetic dissection of Alzheimer's disease and related dementias: amyloid and its relationship to tau. *Nature Neurosci* 1, 355-358 (1998).
Hollosi, M., Otvos, L., Kaijtar, J., Percell, A. and Lee, V.M.Y. Is amyloid deposition in Alzheimer's disease preceded by an environment induced double conformational transition? *Pept Res* 2, 109-113 (1989).
Hoogenboom, H.R., de Bruine, A.P., Hufton, S.E., Hoet, R.M. Arends, J.W. and Roovers, R.C. Antibody phage display technology and its applications. *Immunotechnology* 4:1-20 (1998).
Howlett, D.R., Jennings, K.H., Lee, D.C., Clark, M.S.G., Brown, F., Wetzel, R., Wood, S.J.,Camilleri, P. and Roberts, G.W. Aggregation state and neurotoxic properties of Alzheimer beta-amyloid peptide. *Neurodegeneration,* 4, 23-32 (1995).
Janus, C., Pearson, J., McLaurin, J., Mathews, P.M. Jiang, Y., Schmidt, S.D., Azhar Chisti, M., Horne, P., Heslin, D., French, J., Mount, H.T.J., Nixon, R.A., Mercken, M., Bergeron, C., Fraser, P.E., St. George-Hyslop, P. and Westaway, D. Aβ peptide immunization reduces behavioural impairment and plaques in a model of Alzheimer's disease. *Nature* 408:979-982 (2000).
Karplus, M. and Petsko, G.A. Molecular dynamics simulations in biology. *Nature,* 347, 631-639 (1990).
Katzav-Gozansky, T., Hanan, E. and Solomon, B. Effect of monoclonal antibodies in preventing carboxypeptidase A aggregation. *Biotechnol Appl Biochem* 23,227-230 (1996).
Kelly, J.W. Alternative conformations of amyloidogenic proteins govern their behavior. *Curr. Opin. Struct. Biol*. 6, 11-17 (1996).
Kensil et al., Vaccine Design: The Subunit and Adjuvant Approach (1995)
Kirshenbaum, K. and Daggett, V. PH-Dependent conformations of the amyloid β (1-28) peptide fragment explored using molecular dynamics. *Biochemistry* 34, 7629-7639 (1995).
Langer, *Science,* 249:1527 (1990)
Levy-Lahad, E., Wasco, W., Poorkaj, P. et al. Candidate gene for the chromosome 1 familial Alzheimer's disease locus. *Science* 269, 973-977 (1995).
Lorenzo, A. and Yankner, B.A. β-Amyloid neurotoxicity requires fibril formation and is inhibited by Congo red. *Proc. Natl. Acad. Sci. USA* 91, 12243-12247 (1994).
Maggio, J.E. and Mantyh, P.W. Brain amyloid - a physicochemical perspective. *Brain Pathol* 6, 147-162 (1996).
Medynski, D. Phage display: all dressed up and ready to role. *Biol Technol* 12, 1134-1136 (1994).
Meola, A., Delmastro, P., Monaci, P. et al. Derivation of vaccines from mimotopes: Immunological properties of human B virus surface antigen mimotopes displayed on filamentous phage. *J Immuno* 154, 3162-3172 (1995).
Morgan, D., Diamond, D.M., Gottschall, P.E., Ugen, K.E., Dickey, C., Hardy, J., Duff, K., Jantzen, P., Dicarlo, G., Wilcock, D., Connor, K., Hatcher, J., Hope, C., Gordon, M. and Arendash, G.W. Aβ peptide vaccination prevents memory loss in an animal model of Alzheimer's disease. *Nature* 408:982-985 (2000).
Murphy, C. Loss of olfactory function in dementing disease. *Physiol. Behav.* 66, 177-182 (1999).
Pike, C.J., Overman, M.J. and Cotman, C.W. Amino-terminal deletions enhance aggregation of β-amyloid peptides *in vitro. J Biol Chem* 270, 23895-23898 (1995).
Price, D.L., Borchelt, D.R. and Sisodia, S.S. Alzheimer disease and prion disorders amyloid β-protein and prion protein amyloidoses. *Proc Natl Acad Sci USA,* 90, 6381-6384 (1993)
Schenk, D., Barbour, R., Dunn, W., Gordon, G., Grajeda, H., Guido, T., Hu, K., Huang, J., Johnson-Wood, K., Khan, K., Kholodenko, D., Lee, M., Zhenmei, L., Lieberburg, I., Motter, R., Mutter, L., Soriano, F., Shopp, G., Vasquez, N., Vandevert, C., Walker, S., Wogulis, M., Yednock, T., Games, D. and Seubert, P. Immunization with amyloid-β attenuates Alzheimer'disease-like pathology in the PDAPP mouse. *Nature,* 400, 173-177 (1999).
Scott, J.K. and Smith, G.P. Searching for peptide ligands with an epitope library. *Science*, 249, 386-390 (1990).
Selkoe, D.J. Amyloid β-protein and the genetics of Alzheimer's disease. *J Biol Chem.* 271, 18295-18298 (1996).
Sherrington, R., Rogaev, E.I., Liang, Y. et al. Cloning of a gene bearing missense mutations in early-onset familial Alzheimer's disease. *Nature* 375, 754-760 (1995).
Silen, J.L. and Agard, D.A. The x-yltic protease pro-region does not require a physical linkage to activate the protese domain *in vivo. Nature* 341, 462-464 (1989).
Sladowski, D., Steer, S.J., Clothier, R.H. and Balls, M. An improved MTT assay. *J Immunol Methods.* 157, 203-207 (1993).
Solomon, B. and Balas, N. Thermostabilization of Carboxypeptidase A by interaction with its monoclonal antibodies. *Biotechnol and App Biochem* 14, 202-211 (1991).
Solomon, B. and Schwartz, F. Chaperone-like effect of monoclonal antibodies on refolding of heat-denatured carboxypeptidase A. *J of Molec Recognition.* 8, 72-76 (1995).
Solomon, B., Koppel, R., Hannan, E. and Katzav, T. Monoclonal antibodies inhibit *in vitro* fibrillar aggregation of the Alzheimer's β-amyloid peptide. *Proc Natl Acad Sci USA,* 93, 452455 (1996).
Solomon, B., Koppel, R., Frankel, D. and Hanan-Aharon, E. Disaggregation of Alzheimer beta-amyloid by site-directed mAb. *Proc Natl Acad Sci USA* 94, 4109-4112 (1997).
Solomon, B. and Frenkel, D. Vaccination for the prevention and treatment of Alzheimer's disease. *Drugs of Today,* 36(9), 655-663 (2000).
Soto, C. Alzheimer's and prion disease as disorders of protein conformation: implications for the design of novel therapeutic approaches. *J*. *Mol. Med.* 77, 412-418 (1999).
Soto, C., Castano, E.M., Frangione, B. and Inestrosa, N.C. The α-helical to β-strand transition in the amino-terminal fragment of the amyloid β-peptide modulates amyloid formation. *J Biol Chem* 270, 3063 (1995).
Stoute et al., *N. Engl. J. Med.,* 336:86-91 (1997)
Tam et al., *J*. *Am. Chem. Sic.,* 105:6442 (1983)
Tam et al., *J*. *Am. Chem. Soc.,* 108:5242 (1986)
Van Leuven, F. Single and multiple transgenic mice as models for Alzheimer's disease. *Prog Neurobiol.* 200061(3):305-12. Review (2000).
Willis, E.A., Perham, N.R. and Wraaith, D. (1993). Immunological properties of foreign peptides in multiple display on a filamentous bacteriophage. Gene, 128, 79-83.
Winter, G., Griffiths, A.D., Hawkins, R.E. and Hoogenboom, H.R. Making antibody by phage display technology. *Annu Rev Immunol* 12:433-455 (1994).
Zuercher, A.W., Miescher, S.M., Voge, M., Rudolf, M.P., Stadler, M.B. and Stadler, B.M. Oral anti-IgE immunization with epitope-displaying phage. *Eur. J*. *Immunol.* 30, 128-135 (2000).

## Claims

1. An antigenic product containing a plurality of amyloid-β epitopes, comprising a dendritic polymer, built on a core molecule which is at least difunctional so as to provide branching and containing up to 16 terminal functional groups to each of which an antigenic peptide that comprises an epitope of amyloid β comprising the amino acid sequence of SEQ ID NO:5 is joined by covalent bonds.

2. The antigenic product of claim 1, wherein said dendritic polymer contains eight terminal functional groups to which an antigenic peptide is joined.

3. The antigenic product of claim 1, wherein said dendritic polymer contains four terminal functional groups to which an antigenic peptide is joined.

4. The antigenic product of claim 1, wherein said dendritic polymer contains 16 terminal functional groups to which an antigenic peptide is joined.

5. The antigenic product of any one of claims 1 to 4, wherein said antigenic peptide comprises the amino acid sequence of SEQ ID NO:1.

6. The antigenic product of any one of claims 1 to 5, wherein said core molecule is lysine.

7. The antigenic product of any one of claims 1 to 5, wherein said core molecule is selected from the group consisting of aspartic acid and glutamic acid.

8. The antigenic product of any one of claims 1 to 5, wherein said core molecule has the formula: 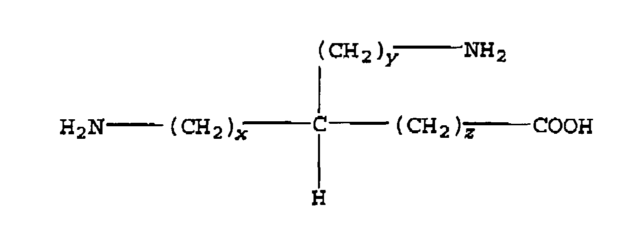 wherein x, y and z are integers from 0 to 10 and at least one of x, y or z is 1.

9. The antigenic product of claim 8, wherein the integers x, y, and z sums up to a total in a range from 2 to 6 and the amino groups are separated by at least two methylene groups.

10. The antigenic product of claim 8, wherein said core molecule is selected from the group consisting of ornithine, nor-lysine, and amino alanine.

11. The antigenic product of any one of claims 1 to 5, wherein said core molecule has the formula:
H₂N-CH₂- (CH₂)ₙ-CH₂-NH₂
wherein n is an integer from 0 to 10.

12. The antigenic product of any one of claims 1 to 11, wherein the antigenic peptide comprises two epitopes of said deposit-forming polypeptide.

13. The antigenic product of claim 12, wherein said two epitopes are identical.

14. The antigenic product of claim 13, wherein the antigenic peptide comprises the amino acid sequence of SEQ ID NO:4.

15. The antigenic product of any one of claims 1 to 14, further comprising an avidin or streptavidin molecule, wherein one to four of said dendritic polymers are each bound to said avidin or streptavidin molecule via a biotin molecule conjugated to said dendritic polymer to form a complex with said avidin or streptavidin molecule.

16. The antigenic product of claim 15, wherein two or three of said dendritic polymers are bound to said avidin or streptavidin molecule.

17. The antigenic product of any one of claims 1 to 16, further comprising a molecule having adjuvant properties joined to said dendritic polymer.

18. The antigenic product of any one of claims 1 to 17, which is encapsulated in a liposome.

19. An immunizing composition, comprising the antigenic product of any one of claims 1 to 18 and a pharmaceutically acceptable carrier, excipient, adjuvant, or auxiliary agent.

20. The use of an antigenic product according to any one of claims 1 to 18 for producing therapeutics for the treatment of a plaque-forming disease or disorder involving amyloid β as the deposit-forming polypeptide.

21. The use of claim 20, wherein said plaque-forming disease or disorder is Alzheimer's disease.

## Patentansprüche

1. Antigenisches Produkt, umfassend eine Vielzahl von Amyloid-β-Epitopen, umfassend ein dendritisches Polymer, das auf einem Gerüstmolekül aufgebaut ist, welches zumindest bifunktionell ist, so dass es Verzweigungen erlaubt und bis zu 16 endständige funktionelle Gruppen aufweist, die jeweils mit einem antigenischen Peptid durch kovalente Bindungen verbunden sind, wobei das antigenische Peptid ein Epitop des Amyloid β aufweist, welches die Aminosäuresequenz SEQ ID NO:5 beinhaltet.

2. Antigenisches Produkt gemäß Anspruch 1, wobei das dendritische Polymer acht endständige funktionelle Gruppen aufweist, an die ein antigenisches Peptid gebunden ist.

3. Antigenisches Produkt gemäß Anspruch 1, wobei das dendritische Polymer vier endständige funktionelle Gruppen aufweist, an die ein antigenisches Peptid gebunden ist.

4. Antigenisches Produkt gemäß Anspruch 1, wobei das dendritische Polymer 16 endständige funktionelle Gruppen aufweist, an die ein antigenisches Peptid gebunden ist.

5. Antigenisches Produkt gemäß mindestens einem der Ansprüche 1 bis 4, wobei das antigenische Peptid die Aminosäuresequenz SEQ ID NO: 1 aufweist.

6. Antigenisches Produkt gemäß mindestens einem der Ansprüche 1 bis 5, wobei das Gerüstmolekül Lysin ist.

7. Antigenisches Produkt gemäß mindestens einem der Ansprüche 1 bis 5, wobei das Gerüstmolekül aus der Gruppe ausgewählt ist, die aus Asparaginsäure und Glutaminsäure besteht.

8. Antigenisches Produkt gemäß mindestens einem der Ansprüche 1 bis 5, wobei das Gerüstmolekül die Formel aufweist: 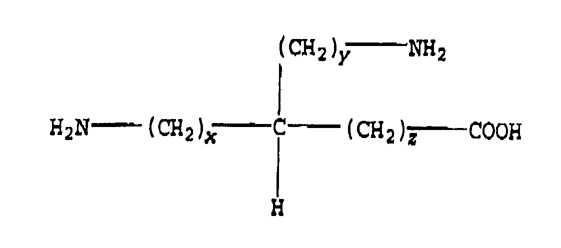 wobei x, y und z ganze Zahlen von 0 bis 10 sind und zumindest eine Zahl von x, y oder z gleich 1 ist.

9. Antigenisches Produkt gemäß Anspruch 8, wobei die ganzen Zahlen x, y und z sich zu einer Summe im Bereich von 2 bis 6 addieren und die Aminogruppen zumindest durch zwei Methylengruppen getrennt sind.

10. Antigenisches Produkt gemäß Anspruch 8, wobei das Gerüstmolekül aus der Gruppe ausgewählt ist, die aus Ornithin, Nor-Lysin und Aminoalanin besteht.

11. Antigenisches Produkt gemäß mindestens einem der Ansprüche 1 bis 5, wobei das Gerüstmolekül folgende Formel aufweist:
H₂N-CH₂(CH₂)ₙ-CH₂-NH₂,
wobei n eine ganze Zahl von 0 bis 10 ist.

12. Antigenisches Produkt gemäß mindestens einem der Ansprüche 1 bis 11, wobei das antigenische Peptid zwei Epitope des Polypeptids, das Ablagerungen bildet, aufweist.

13. Antigenisches Produkt gemäß Anspruch 12, wobei die beiden Epitope identisch sind.

14. Antigenisches Produkt gemäß Anspruch 13, wobei das antigenische Peptid die Aminosäuresequenz SEQ ID NO:4 umfasst.

15. Antigenisches Produkt gemäß mindestens einem der Ansprüche 1 bis 14, welches zusätzlich ein Avidin- oder Streptavidin-Molekül aufweist, wobei eines bis vier der dendritischen Polymere jeweils an das Avidin- oder Streptavidin-Molekül durch ein Biotinmolekül verbunden ist/sind, so dass das dendritische Polymer mit dem Avidin- oder Streptavidin-Molekül einen Komplex bildet.

16. Antigenisches Produkt gemäß Anspruch 15, wobei zwei oder drei der dendritischen Polymere an das Avidin- oder Streptavidin-Molekül gebunden sind.

17. Antigenisches Produkt gemäß mindestens einem der Ansprüche 1 bis 16, das zusätzlich ein Molekül mit Eigenschaften eines Adjuvans aufweist, welches mit dem dendritischen Polymer verbunden ist.

18. Antigenisches Produkt gemäß mindestens einem der Ansprüche 1 bis 17, welches in einem Liposom verkapselt ist.

19. Immunisierende Zusammensetzung, die das antigenische Produkt gemäß mindestens einem der Ansprüche 1 bis 18 aufweist und einen pharmazeutisch geeigneten Träger, einen Arzneistoffträger, ein Adjuvans, oder einen Hilfsstoff umfasst.

20. Verwendung eines antigenischen Produkts gemäß mindestens einem der Ansprüche 1 bis 18 zur Herstellung von Therapeutika zur Behandlung bei Erkrankung, die zur Bildung von Ablagerungen führt, oder einer Störung, die Amyloid β als Polypeptid, das Ablagerungen bildet, einschließt.

21. Verwendung gemäß Anspruch 20, wobei es sich bei der Krankheit, bei der Ablagerungen gebildet werden, oder der Störung um die Alzheimersche Erkrankung handelt.

## Revendications

1. Produit antigénique comprenant une pluralité d'épitopes d'amyloïde-β, comprenant un polymère dendritique, construit sur une molécule centrale qui est au moins difonctionnelle de manière à fournir une ramification et contenant jusqu'à 16 groupes fonctionnels terminaux, auxquels chaque peptide antigénique qui comprend un épitope d'amyloïde-β comprenant la séquence d'aminoacides de la SEQ ID N° 5 est joint par des liaisons covalentes.

2. Produit antigénique suivant la revendication 1, dans lequel ledit polymère dendritique contient huit groupes fonctionnels terminaux auxquels un peptide antigénique est joint.

3. Produit antigénique suivant la revendication 1, dans lequel ledit polymère dendritique contient quatre groupes fonctionnels terminaux auxquels un peptide antigénique est joint.

4. Produit antigénique suivant la revendication 1, dans lequel ledit polymère dendritique contient seize groupes fonctionnels terminaux auxquels un peptide antigénique est joint.

5. Produit antigénique suivant l'une quelconque des revendications 1 à 4, ledit peptide antigénique comprenant la séquence d'aminoacides de la SEQ ID N° 1.

6. Produit antigénique suivant l'une quelconque des revendications 1 à 5, dans lequel ladite molécule centrale est la lysine.

7. Produit antigénique suivant l'une quelconque des revendications 1 à 5, dans lequel ladite molécule centrale est choisie dans le groupe consistant en l'acide aspartique et l'acide glutamique.

8. Produit antigénique suivant l'une quelconque des revendications 1 à 5, dans lequel ladite molécule centrale répond à la formule : 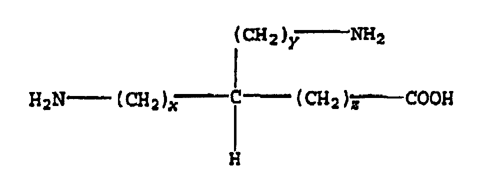 dans laquelle x, y et z représentent des nombres entiers de 0 à 10 et au moins l'un de x, y et z est égal à 1.

9. Produit antigénique suivant la revendication 8, dans lequel la somme des nombres entiers x, y et z va jusqu'à une valeur totale de 2 à 6 et les groupes amino sont séparés par au moins deux groupes méthylène.

10. Produit antigénique suivant la revendication 8, dans lequel ladite molécule centrale est choisie dans le groupe consistant en l'ornithine, la norlysine et l'aminoalanine.

11. Produit antigénique suivant l'une quelconque des revendications 1 à 5, dans lequel ladite molécule centrale répond à la formule :
H₂N-CH₂- (CH₂)ₙ-CH₂-NN₂
dans laquelle n représente un nombre entier de 0 à 10.

12. Produit antigénique suivant l'une quelconque des revendications 1 à 11, ledit peptide antigénique comprenant deux épitopes dudit polypeptide formant un dépôt.

13. Produit antigénique suivant la revendication 12, dans lequel lesdits deux épitopes sont identiques.

14. Produit antigénique suivant la revendication 13, le peptide antigénique comprenant la séquence d'aminoacides de la SEQ ID N° 4.

15. Produit antigénique suivant l'une quelconque des revendications 1 à 14, comprenant en outre une molécule d'avidine ou de streptavidine, dans lequel un à quatre desdits polymères dendritiques sont chacun liés à ladite molécule d'avidine ou de streptavidine par l'intermédiaire d'une molécule de biotine conjuguée audit polymère dendritique pour former un complexe avec ladite molécule d'avidine ou de streptavidine.

16. Produit antigénique suivant la revendication 15, dans lequel deux ou trois desdits polymères dendritiques sont liés à ladite molécule d'avidine ou de streptavidine.

17. Produit antigénique suivant l'une quelconque des revendications 1 à 16, comprenant en outre une molécule ayant des propriétés d'adjuvant jointe audit polymère dendritique.

18. Produit antigénique suivant l'une quelconque des revendications 1 à 17, qui est encapsulé dans un liposome.

19. Composition immunisante, comprenant le produit antigénique de l'une quelconque des revendications 1 à 18 et un support, excipient, adjuvant ou agent auxiliaire pharmaceutiquement acceptable.

20. Utilisation d'un produit antigénique suivant l'une quelconque des revendications 1 à 18 pour la production d'agents thérapeutiques destinés au traitement d'une maladie ou d'un trouble formant des plaques impliquant l'amyloïde-β comme polypeptide formant un dépôt.

21. Utilisation suivant la revendication 20, dans laquelle ladite maladie ou ledit trouble formant des plaques est la maladie d'Alzheimer.


### SEQUENCE LISTING

<110> SOLOMON, Beka
<120> ANTIGENIC PRODUCT DISPLAYING MULTIPLE COPIES OF AN EPITOPE OF A DEPOS IT-FORMING POLYPEPTIDE INVOLVED IN PLAQUE-FORMING DISEASES AND METHODS OF US ING SAME
<130> SOLOMON=4.1A PCT
<140> NOT YET ASSIGNED
   <141> 2002-06-20
<160> 7
<170> PatentIn version 3.1
<210> 1
   <211> 8
   <212> PRT
   <213> Artificial Sequence
<220>
   <223> Synthetic
<400> 1
   Tyr Tyr Glu Phe Arg His Asp Ser
   1               5
<210> 2
   <211> 9
   <212> PRT
   <213> Artificial Sequence
<220>
   <223> Synthetic
<400> 2
   Asp Ala Glu Phe Arg His Asp Ser Gly
   1               5
<210> 3
   <211> 4
   <212> PRT
   <213> Artificial Sequence
<220>
   <223> Synthetic
<400> 3
   Trp Val Leu Asp
   1
<210> 4
   <211> 10
   <212> PRT
   <213> Artificial Sequence
<220>
   <223> Synthetic
<400> 4
   Tyr Glu Phe Arg His Tyr Glu Phe Arg His
   1               5                   10
<210> 5
   <211> 4
   <212> PRT
   <213> Artificial Sequence
<220>
   <223> Synthetic
<400> 5
   Glu Phe Arg His
   1
<210> 6
   <211> 9
   <212> PRT
   <213> Artificial
<220>
   <223> Synthetic
<400> 6
   Asp Tyr Glu Asp Arg Tyr Tyr Arg Glu
   1               5
<210> 7
   <211> 9
   <212> PRT
   <213> Artificial
<220>
   <223> Synthetic
<400> 7
   Asp Trp Glu Asp Arg Tyr Tyr Arg Glu
   1               5